# EUROPÄISCHE PATENTSCHRIFT

(11) **EP 1 383 773 B1**
(45) Veröffentlichungstag und Bekanntmachung des Hinweises auf die Patenterteilung: **29.12.2004**
(21) Anmeldenummer: 02732621.4
(22) Anmeldetag: 16.04.2002
(51) Int. Cl.: C07D 513/04, A01N 43/90

(54) **NEUE INSEKTIZID WIRKENDE AZOLE**
NOVEL INSECTICIDAL AZOLES
AZOLES A EFFET INSECTICIDE

(30) Priorität: 20.04.2001 DE 10119422
(43) Veröffentlichungstag der Anmeldung: 28.01.2004
(73) Patentinhaber: Bayer CropScience AG, 40789 Monheim (DE)
(72) Erfinder: JESCHKE, Peter, 51467 Bergisch Gladbach (DE); BECK, Michael, 41363 Jüchen (DE); KRÄMER, Wolfgang, 51399 Burscheid (DE); WOLLWEBER, Detlef, 42113 Wuppertal (DE); LUBOS-ERDELEN, Angelika, 42799 Leichlingen (DE); TURBERG, Andreas, 42781 Haan (DE); HANSEN, Olaf, 42799 Leichlingen (DE); MARTIN, Hans-Dieter, 40591 Düsseldorf (DE); SAUER, Piet, 89518 Heidenheim (DE)
(86) Internationale Anmeldenummer: PCT/EP2002/004176
(87) Internationale Veröffentlichungsnummer: WO 2002/085915

(56) Entgegenhaltungen:
- EP-A- 0 259 738
- EP-A- 0 315 826

## Beschreibung

Die vorliegende Erfindung betrifft neue heterocyclische Verbindungen, Verfahren zu ihrer Herstellung und ihrer Verwendung als Pflanzenschutzmittel, insbesondere zur Bekämpfung von tierischen Schädlingen.

Verschiedene, substituierte Imino-Bicyclen, wie beispielsweise das 6-(2-Methylaminoeihylimino)-2,3,4,8-tetrahydro-6H-thiazolo[3,4-a]pyrimidin-Hydrochlorid, sind bereits als pharmakologisch wirksame Verbindungen mit bluthochdrucksenkender Aktivität bekannt geworden. Über ihre Verwendung als Pflanzenschutzmittel und insbesondere zur Bekämpfung von tierischen Schädlingen ist jedoch nichts bekannt geworden (vgl. US-Pat. 3,578,666).

Weitere substituierte Imino-Bicyclen, wie beispielsweise die 5-Imino-2,3-dihydroimidazo[1,2-c]thiazole sind bekannt (vgl. US-Pat. 3,555,039). Bestimmte Imino-Bicyclen dieses Strukturtyps, wie beispielsweise das 7-Phenyl-5-imino-2,3-dihydro-1H,5H-imidazo[1,2-c]thiazol-Hydrochlorid, zeigen bluthochrucksenkende und das Zentralnervensystem stimulierende Aktivität. Über die Verwendung als Pflanzenschutzmittel, insbesondere zur Bekämpfung von tierischen Schädlingen, ist jedoch nichts bekannt geworden.

Es wurden nun neue heterocyclische Verbindungen der Formel (I) gefunden, 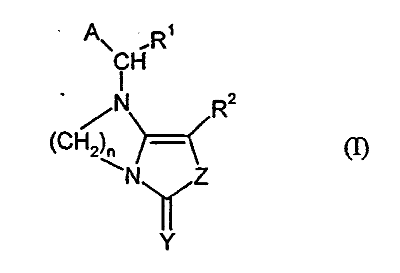 in welcher
- A: für jeweils gegebenenfalls substituiertes Aryl oder Hetaryl oder Heterocyclyl steht,
- R¹: für Wasserstoff oder C₁-C₃-Alkyl steht,
- R²: für Wasserstoff, C₁-C₃-Alkyl oder jeweils gegebenenfalls substituiertes Aryl oder Hetaryl steht,
- n: für 2, 3 oder 4 steht,
- Y: für N-CN oder N-NO₂ steht,
- Z: für S, SO, SO₂ oder NR³ steht und
- R³: für Wasserstoff oder C₁-C₃-Alkyl steht.

Weiterhin wurde gefunden, dass man die Verbindungen der Formel (I) erhält, wenn man Verbindungen der Formel (II) 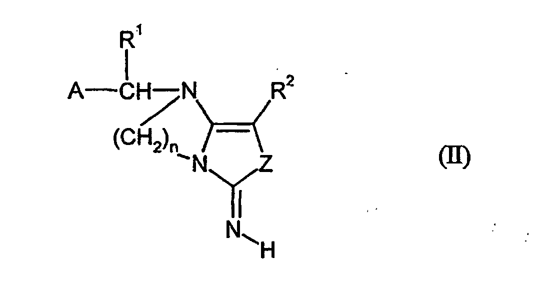 mit geeigneten Cyanierungsreagenzien (zum Erhalt von Verbindungen der Formel (T) in welchen Y für N-CN steht) oder mit geeigneten Nitrierungsreagenzien (zum Erhalt von Verbindungen der Formel (1) in welchen Y für N-NO₂ steht) umsetzt.

Schließlich wurde gefunden, dass die neuen Verbindungen der Formel (I) stark ausgeprägte biologische Eigenschaften besitzen und vor allem zur Bekämpfung von tierischen Schädlingen, insbesondere von Insekten, Spinnentieren und Nematoden, die in der Landwirtschaft, in den Forsten, im Vorrats- und Materialschutz sowie auf dem Hygienesektor vorkommen, geeignet sind.

Die erfindungsgemäßen Verbindungen sind durch die Formel (I) allgemein definiert.

Bevorzugte Substituenten bzw. Bereiche der in den oben und nachstehend erwähnten Formeln aufgeführten Reste werden im folgenden erläutert.
- A: steht bevorzugt für gegebenenfalls durch Halogen, Cyano, Nitro, C₁-C₄-Alkyl, C₁-C₄-Halogenalkyl, C₁-C₄-Alkoxy oder C₁-C₄-Halogenalkoxy substituiertes Phenyl.
- A: steht bevorzugt weiterhin für Pyrazolyl, 1,2,4-Triazolyl, Oxazolyl, Isoxazolyl, Thiazolyl, Isothiazolyl, 1,2,5-Thiadiazolyl, Pyridyl, Pyrazinyl oder Pyrimidinyl, welche gegebenenfalls durch Fluor, Chlor, Brom, Cyano, Nitro, C₁-C₂-Alkyl (welches gegebenenfalls durch Fluor und/oder Chlor substituiert ist), C₁-C₂-Alkoxy (welches gegebenenfalls durch Fluor und/oder Chlor substituiert ist), C₁-C₂-Alkylthio (welches gegebenenfalls durch Fluor und/oder Chlor substituiert ist) oder C₁-C₂-Alkylsulfonyl (welches gegebenenfalls durch Fluor und/oder Chlor substituiert ist) substituiert sind.
- A: steht weiterhin für einen gegebenenfalls durch Halogen oder C₁-C₃-Alkyl substituierten gesättigten C₅-C₆-Cycloalkylrest, in welchem eine Methylengruppe durch O oder S ersetzt ist.
- R¹: steht bevorzugt für Wasserstoff, Methyl, Ethyl, n-Propyl oder i-Propyl.
- R²: steht bevorzugt für Wasserstoff, Methyl, Ethyl, n-Propyl, i-Propyl, für gegebenenfalls durch Halogen, Cyano, Nitro, C₁-C₄-Alkyl, C₁-C₄-Halogenalkyl, C₁-C₄-Alkoxy oder C₁-C₄-Halogenalkoxy substituiertes Phenyl oder für Pyrazolyl, 1,2,4-Triazolyl, Oxazolyl, Isoxazolyl, Thiazolyl, Isothiazolyl, 1,2,5-Thiadiazoiyl, Pyridyl, Pyrazinyl oder Pyrimidinyl, welche gegebenenfalls durch Fluor, Chlor, Brom, Cyano, Nitro, C₁-C₂-Alkyl (welches gegebenenfalls durch Fluor und/oder Chlor substituiert ist), C₁-C₂-Alkoxy (welches gegebenenfalls durch Fluor und/oder Chlor substituiert ist), C₁-C₂-Alkylthio (welches gegebenenfalls durch Fluor und/oder Chlor substituiert ist) oder C₁-C₂-Alkylsulfonyl (welches gegebenenfalls durch Fluor und/oder Chlor substituiert ist) substituiert sind.
- n: steht bevorzugt für 2, 3 oder 4 (insbesondere für 2 oder 3).
- Y: steht bevorzugt für N-CN oder N-NO₂.
- Z: steht bevorzugt für S oder NR³.
- R³: steht bevorzugt für Wasserstoff, Methyl, Ethyl, n-Propyl oder i-Propyl.
- A: steht besonders bevorzugt für Thiazolyl oder Pyridyl welche jeweils gegebenenfalls substituiert sind durch Halogen (insbesondere Chlor) oder C₁-C₃-Alkyl (insbesondere Methyl).
- A: steht weiterhin besonders bevorzugt für einen gegebenenfalls durch Halogen (insbesondere Chlor) oder C₁-C₃-Alkyl (insbesondere Methyl) substituierten Tetrahydrofurylrest.
- R¹: steht besonders bevorzugt für Wasserstoff oder Methyl.
- R²: steht besonders bevorzugt für Wasserstoff, Methyl, oder gegebenenfalls durch Halogen, Cyano oder durch jeweils gegebenenfalls durch Fluor oder Chlor substituiertes Methyl, Ethyl, Methoxy oder Ethoxy substituiertes Phenyl oder für Thiazolyl, Pyridyl oder Pyrazinyl.
- n: steht besonders bevorzugt für 2 oder 3.
- Y: steht besonders bevorzugt für NCN oder NNO₂.
- Z: steht besonders bevorzugt für S oder NR³.
- R³: steht besonders bevorzugt für Wasserstoff oder Methyl.
- A: steht ganz besonders bevorzugt für einen der Reste 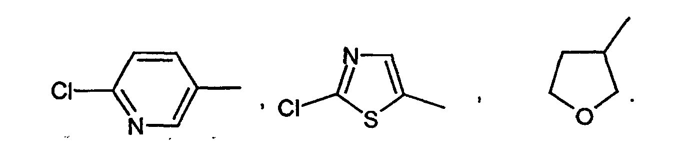
- R¹: steht ganz besonders bevorzugt für Wasserstoff oder Methyl, insbesondere für Wasserstoff.
- R²: steht ganz besonders bevorzugt für Wasserstoff oder Methyl.
- n: steht ganz besonders bevorzugt für 2 oder 3.
- Y: steht ganz besonders bevorzugt für NCN.
- Y: steht weiterhin ganz besonders bevorzugt für NNO₂.
- Z: steht ganz besonders bevorzugt für S oder NR³.
- R³: steht ganz besonders bevorzugt für Wasserstoff oder Methyl.

In einer hervorgehobenen Gruppe von Verbindungen der Formel (I) steht n für 2.

In einer weiteren hervorgehobenen Gruppe von Verbindungen der Formel (I) steht n für 3.

In einer weiteren hervorgehobenen Gruppe von Verbindungen der Formel (I) steht Y für N-CN.

In einer weiteren hervorgehobenen Gruppe von Verbindungen der Formel (I) steht Y für N-NO₂.

In einer weiteren hervorgehobenen Gruppe von Verbindungen der Formel (I) steht A für

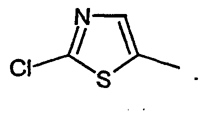

In einer weiteren hervorgehobenen Gruppe von Verbindungen der Formel (I) steht A für

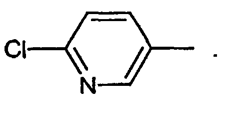

In einer weiteren hervorgehobenen Gruppe von Verbindungen der Formel (I) steht A für

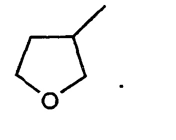

Die oben aufgeführten allgemeinen oder in Vorzugsbereichen aufgeführten Restedefinitionen bzw. Erläuterungen gelten für die Endprodukte und für die Ausgangsund Zwischenprodukte entsprechend. Diese Restedefinitionen können untereinander, also auch zwischen den jeweiligen Vorzugsbereichen, beliebig kombiniert werden.

Erfindungsgemäß bevorzugt werden die Verbindungen der Formel (1), in welchen eine Kombination der vorstehend als bevorzugt aufgeführten Bedeutungen vorliegt.

Erfindungsgemäß besonders bevorzugt werden die Verbindungen der Formel (I), in welchen eine Kombination der vorstehend als besonders bevorzugt aufgeführten Bedeutungen vorliegt.

Erfindungsgemäß ganz besonders bevorzugt werden die Verbindungen der Formel (I), in welchen eine Kombination der vorstehend als ganz besonders bevorzugt aufgeführten Bedeutungen vorliegt.

In den oben und nachstehend aufgeführten Restedefinitionen sind Kohlenwasserstoffreste, wie Alkyl - auch in Verbindung mit Heteroatomen wie in Alkoxy - soweit möglich jeweils geradkettig oder verzweigt.

Verwendet man beispielsweise unten gezeigte Verbindungen der Struktur A und Bromcyan als Ausgangsstoffe, so kann der Verlauf des erfindungsgemäßen Verfahrens durch das folgende Reaktionsschema wiedergegeben werden:

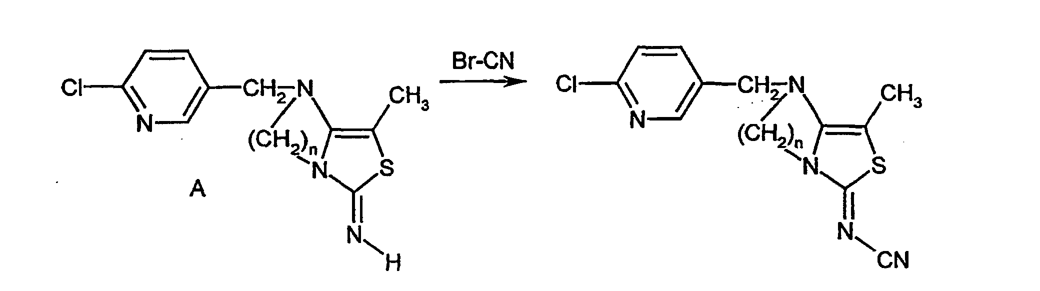

Die Verbindungen der Formel (II) sind neu und auch Gegenstand der vorliegenden Erfindung.

In Abhängigkeit insbesondere von der Bedeutung der Variablen Z kommen für die Herstellung der Verbindungen der Formel (II) verschiedene Methoden in Betracht Auch einige Verbindungen der Formel (I) lassen sich nach weiteren Methoden herstellen.

Zur Erläuterung soll zunächst das folgende Formelschema 1 dienen.

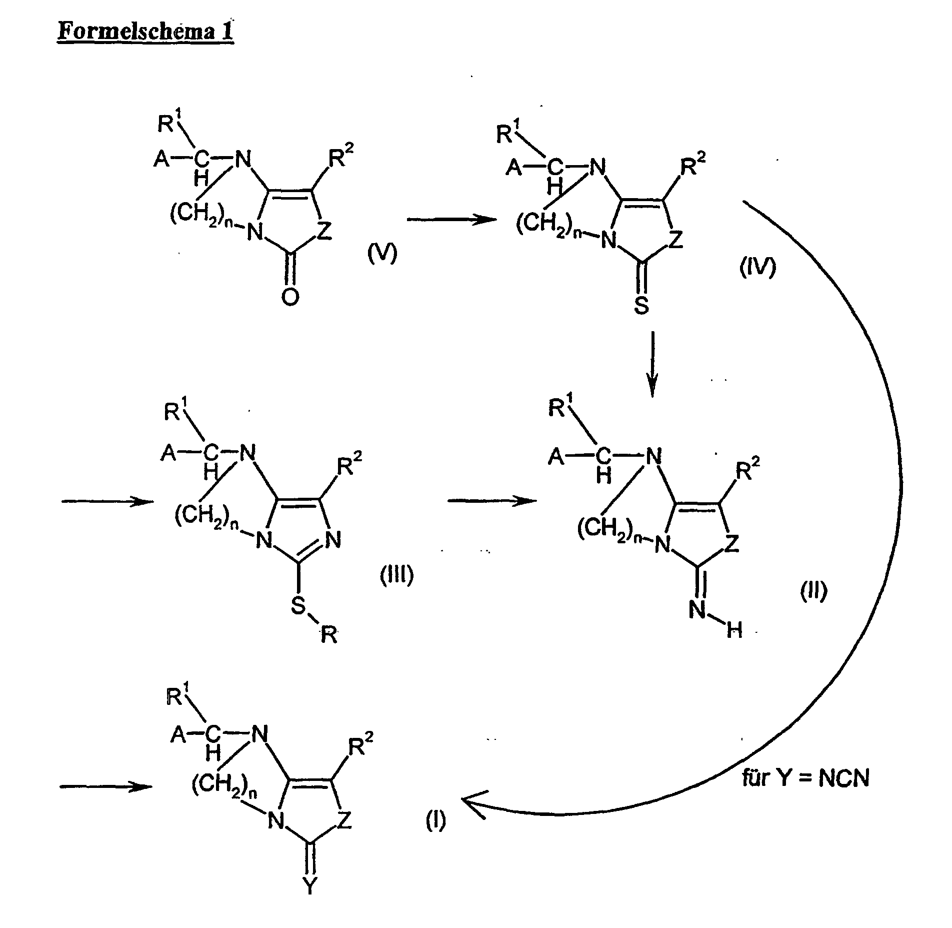

Die im Formelschema 1 beschriebenen Reaktionen lassen sich in allgemein bekannter Weise durchführen.

Die Verbindungen der Formel (V) lassen sich durch Umsetzung mit Schwefelungsreagenzien in die Verbindungen der Formel (IV) überführen.

Eine Vielzahl unterschiedlicher Schweflungsreagenzien sind in der Literatur beschrieben, wie beispielsweise Schwefelwasserstoff (H₂S), Schwefelwasserstoff/Chlorwasserstoff (H₂S/HCl), Wasserstoffpersulfid/Chlorwasserstoff (H₂S₂/HCI), Di-(diethylaluminium)-sulfid [(Et₂Al)₂S], Polymeres Ethylaluminiumsulfid [(EtAlS)ₙ], Siliziumdisulfid (SiS₂), Dibortrisulfid (B₂S₃), Phosphorpentachlorid/Dialuminiumtrisulfid/Natriumsulfat (PCl₅/Al₂S₃/Na₂SO₄), Natriumsulfid/Schwefelsäure (Na₂S/H₂SO₄), Diphosphorpentasulfid (P₂S₅), Diphosphorpentasulfid/Pyridin (P₂S₅/Py), Diethylthiocarbamoylchlorid, Diphosphorpentasulfid/Triethylamin (P₂S₅/NEt₃), Diphosphorpentasulfid/n-Butyllithium (P₂S₅/n-BuLi), Diphosphorpentasulfid/Natriumhydrogencarbonat (P₂S₅/NaHCO₃; "Scheeren's Reagens", Bildung von Na²⁺ [P₄S₁₀O]²⁻), Diphosphorpentasulfid/Methanol (P₂S₅/MeOH), SCN-CO-OEt, PSClₓ · (NMe₂)₃₋ₓ (X = 0-3), Bis(tricyclohexylzinn)sulfid/Bortrihalogenid [(C₆H₁₁)₃Sn)S₂+BX₃ (X = Cl, F), EP 0 280 867 (1988), Bis(1,5-cyclooktandiylboryl)sulfid [(9-BBN)₂S] als Schwefelungsreagens oder als Phosphorpentasulfid-Ersatz 2,4-Bis-(methylthio)-1,3,2,4-dithiadiphosphetan-2,4-disulfid "Davy-Reagens Methyl" (DR-Me), 2,4-Bis-(ethylthio)-1,3,2,4-dithiadiphosphetan-2,4-disulfid "Davy-Reagens Ethyl" (DR-Et), 2,4-Bis-(p-tolylthio)-1,3,2,4-dithiadiphosphetan-2,4-disulfid "Davy-Reagens p-Tolyl oder Heimgartner Reagens" (DR-T), 2,4-Bis-(4-phenoxyphenyl)-2,4-dithioxo-1,3,2,4-dithiadiphosphetan "Belleau's Reagens (BR)", 2,4-Bis-(4-phenylthiophenyl)-2,4-dithioxo-1,3,2,4-dithiadiphosphetan, 2,4-Bis-(4-methoxyphenyl)-2,4-dithioxo-1,3,2,4-dithiadiphosphetan "Lawesson's Reagens (LR)" (vgl. Davy-Reagens: H. Heimgartner et al., Helv. Chim. Acta 70, 1987, S. 1001; Belleau's Reagens: Tetrahedron 40, 1984, S. 2047; Tetrahedron 40, 1984, S. 2663; Tetrahedron Letters 24, 1983, S. 3815; I. Thomson et al., Org. Synth. 62, 1984, S. 158 sowie dort zitierte Literatur; D. Brillon Synthetic Commun. 20 (19), 1990, S. 3085 und dort zitierte Literatur; selektive Thionierung von Oligopeptiden: K. Clausen et al., J. Chem. Soc., Perkin Trans I 1984, 785; O. E. Jensen et al., Tetrahedron 41, 1985, S. 5595; Reviews über "Lawesson's Reagenz, (LR)": R. A. Cherkasov et al., Tetrahedron 41, 1985, S. 2567; M. P. Cava et al., Tetrahedron 41, 1985, S. 5061; Diborylsulfid: Liebigs Ann. Chem. 1992, S. 1081 und dort zitierte Literatur; Metzner et al. in Sulfur Reagents in Organic Synthesis, B. Harcourt: London 1994, Academic Press, S. 44-45).

Alternativ sind auch Reaktionsfolgen, wie beispielsweise eine O-Alkylierung mit R₃O⁺BF₄⁻ (R: -Methyl, Ethyl) (H. Meerwein et al., Justus Liebigs Ann. Chem. 641, (1961) S. 1) und anschließende Umsetzung der Intermediate mit wasserfreiem NaSH (R. E. Eibeck, Inorg. Syn. 7, (1963) S. 128), die *in-situ* Bildung von Chlor-iminiumsalzen und nachfolgende Reaktion mit Tetrathiomolybdaten, insbesondere Benzyltriethylammoniumtetramolybdat [(Ph-CH₂-NEt₃)₂MoS₄] (Tetrahedron Lett. 36 (45), 1995, S. 8311) oder Hexamethyldisilathian (TMS₂S) (TMS: Trimethylsilyl; P. L. Fuchs et al., J. Org. Chem. 59, 1994, S. 348) möglich.

Zur Durchführung werden als Sulfidierungsreagenzien bevorzugt Phosphorreagenzien wie beispielsweise Diphosphorpentasulfid (P₂S₅), Diphosphorpentasulfid/Pyridin (P₂S₅/Py), Diphosphorpentasulfid/Triethylamin (P₂S₅/NEt₃), Diphosphorpentasulfid/Natriumhydrogencarbonat (P₂S₅/ NaHCO₃ "Scheeren's Reagens") oder besonders bevorzugt das racemisierungsfreie 2,4-Bis-(4-methoxyphenyl)-2,4-dithioxo-1,3,2,4-dithiadiphosphetan (LR: Lawesson's Reagens) (K. Clausen, M. Thorsen, S.-O. Lawesson Tetrahedron 37, 1981, S. 3635) 2,4-Bis-(4-phenoxyphenyl)-2,4-dithioxo-1,3,2,4-dithiadiphosphetan "Belleau's Reagens (BR)" bzw. 2,4-Bis-(4-phenylihiophenyl)-2,4-dithioxo-1,3,2,4-dithiadiphospheian, eingesetzt.

Im allgemeinen ist es vorteilhaft, dieses Verfahren in Gegenwart von Verdünnungsmitteln durchzuführen. Verdünnungsmittel werden vorteilhaft in einer solchen Menge eingesetzt, dass das Reaktionsgemisch während des ganzen Verfahrens gut rührbar bleibt. Als Verdünnungsmittel zur Durchführung des erfindungsgemäßen Verfahrens kommen alle inerten organischen Lösungsmittel in Frage.

Als Beispiele sind zu nennen: Halogenkohlenwasserstoffe, insbesondere Chlorkohlenwasserstoffe, wie Tetrachlorethylen, Tetrachlorethan, Dichlorpropan, Methylenchlorid, Dichlorbutan, Chloroform, Tetrachlorkohlenstoff, Trichlorethan, Trichlorethylen, Pentachlorethan, Difluorbenzol, 1,2-Dichlorethan, Chlorbenzol, Brombenzol, Dichlorbenzol, Chlortoluol, Trichlorbenzol; Alkohole wie Methanol, Ethanol, Isopropanol, Butanol; Ether wie Ethylpropylether, Methyl-tert-butylether, n-Butylether, Anisol, Phenetol, Cyclohexylmethylether, Dimethylether, Diethylether, Dipropylether, Diisopropylether, Di-n-butylether, Düsobutylether, Diisoamylether, Ethylenglycoldimethylether, Tetrahydrofuran, Dioxan, Dichlordiethylether und Polyether des Ethy-lenoxids und/oder Propylenoxids: Amine wie Trimethyl-, Triethyl-, Tripropyl-, Tributylamin, N-Methyl-morpholin, Pyridin und Tetramethylendiamin, Nitrokohlenwasserstoffe wie Niromethan, Nitroethan, Nitropropan, Nitrobenzol, Chlornitrobenzol, o-Nitrotoluol; Nitrile wie Acetonitril, Propionitril, Butyronitril, Isobutyronitril, Benzonitril, m-Chlorbenzonitril sowie Verbindungen wie Tetrahydrothiophendioxid und Dimethylsulfoxid,. Tetramethylensulfoxid, Dipropylsulfoxid, Benzylmethylsulfoxid, Diisobutylsulfoxid, Dibutylsulfoxid, Diisoamylsulfoxid; Sulfone wie Dimethyl-, Diethyl-, Dipropyl-, Dibutyl-, Diphenyl-, Dihexyl-, Methylethyl, Ethylpropyl-, Ethylisobutyl- und Pentamethylensulfon; aliphatische, cycloaliphatische oder aromatische Kohlenwasserstoffe wie Pentan, Hexan, Heptan, Oktan, Nonan und technische Kohlenwasserstoffe, beispielsweise sogenannte White Spirits mit Komponenten mit Siedepunkten im Bereich beispielsweise von 40°C bis 250°C, Cymol, Benzinfraktionen innerhalb eines Siedepunktintervalles von 70°C bis 190°C, Cyclohexan, Methylcyclohexan, Petrolether, Ligroin, Octan, Benzol, Toluol, Chlorbenzol, Brombenzol, Nitrobenzol, Xylol; Ester wie Methyl-, Ethyl-, Butyl-, Isobutylacetat, sowie Dimethyl-, Dibutyl-, Ethylencarbonat; Amide wie Hexamethylenphosphortriamid, Formamid, N-Methyl-formamid, N,N-Dimethylformamid, N,N-Dipropylformamid, N,N-Dibutylformamid, N-Methyl-pyrrolidin, N-Methyl-caprolactam, 1,3-Dimethyl-3,4,5,6-tetrahydro-2(1H)-pyrimidin, Octylpyrrolidon, Octylcaprolactam, 1,3-Dimethyl-2-imidazolindion, N-Formyl-piperidin, N,N'-1,4-Diformylpiperazin; Ketone wie Aceton, Acetophenon, Methylethylketon, Methylbutylketon.

Selbstverständlich kann man das erfindungsgemäße Verfahren auch in Gemischen der genannten Lösungs- und Verdünnungsmittel durchführen.

Die zu verwendenden Verdünnungsmittel sind vom jeweils eingesetzten Schwefelungsreagens abhängig.

Bevorzugte Verdünnungsmittel zur Thionierung sind jedoch aromatische Kohlenwasserstoffe wie Benzol, Toluol, Chlorbenzol, Brombenzol, Nitrobenzol oder Xylol, Ether wie Ethylpropylether, Methyl-tert-butylether, Anisol, Phenetol, Cyclohexylmethylether, Tetrahydrofuran oder Dioxan.

Die Verbindungen der Formel (IV) lassen sich durch Umsetzung mit HgCl₂/(C₂H₅)₃N/H₂NCN direkt in die Verbindungen der Formel (I) überführen, in denen Y für CN steht (Vgl. Can. J. Chem. 1985, 63, 3089 und auch J. Med. Chem. 1988, 31, 264).

Die Verbindungen der Formel (IV) lassen sich ferner durch Aminolyse mit Ammoniak in Gegenwart von Quecksilbersalzen, durch Erhitzen mit ethanolischer Ammoniaklösung oder durch Reaktion mit wässrigem Ammoniak in Gegenwart von geeigneten Oxidationsmitteln, wie beispielsweise tert-Butylhydroperoxid (TBHP), in Verbindungen der Formel (II] überführen (vgl. M.G. Bock et al., J. Med. Chem. 1988, 31, 264-268; N.W. Jacobsen et al., Aust. J. Chem. 1987, 40 491-499; T. Lindel et al. Tetrahedron Lett. 1997,38(52), 8935-8938).

Die Umsetzung der Verbindungen der Formel (IV) (Z = NH) mit Alkyliodiden in Gegenwart von Basen führt zu Verbindungen der Formel (m) (siehe dazu folgende Literaturstellen: T. Lindel et al., Tetrahedron Lett. 1997, 38(52), 8935-8938 M.G. Bock et al., J. Med. Chem. 1988, 31, 264-268). In den Verbindungen der Formel (III) steht R beispielsweise für Alkyl, bevorzugt für Methyl oder Ethyl. Die Verbindungen der Formel (III) wiederum lassen sich in grundsätzlich bekannter Weise durch Umsetzung mit mit einem Gemisch aus Ammoniak/Ammoniakchlorid in Verbindungen der Formel (II) überführen (vgl. T. Lindel et al. Tetrahedron Lett. 1997, 38(52), 8935-8938).

Die Verbindungen der Formel (II) lassen sich mit Cyanierungsreagenzien bzw. Nitrierungsreagenzien in Verbindungen der Formel (I) überführen.

Als Cyanierungsreagenz kommt beispielsweise Bromcyan (BrCN) in Frage: Die Umsetzung wird in allgemein bekannter Weise durchgeführt (vgl. auch US 4 098 791 und DE 29 16 140).

Nitrierungen sind nach üblichen Verfahren, wie sie beispielsweise in Houben-Weyl, Methoden der Organischen Chemie, Band XI/2 (Georg Thieme Verlag-Stuttgart 1958), S. 99-116 beschrieben sind, durchführbar. Als Nitrierungsreagenzien seien rauchende oder 100 %ige Salpetersäure (Darstellung wasserfreier Salpetersäure vgl. F.D. Chattaway, Soc. 97, 2100 (1910)) gegebenenfalls in Gegenwart von Schwefelsäure (W.J. Middleton et al., J. Heterocyclic Chem. 7, 1045-1049 (1970); L.W. Deady et al. Aust J. Chem. 35 (10), 2025-2034 (1982); EP 0 192 060) oder die Verwendung von Salpetersäureestern, Acetylnitrat oder Nitroniumtetrafluoroborat genannt. Bevorzugt wird die Umsetzung mit Acylnitrat in allgemein bekannter Weise durchgeführt.

Verbindungen der Formel (V) 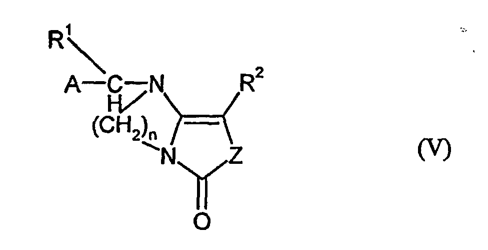 in welcher
A, Z, n, R¹ und R² die oben angegebenen Bedeutungen haben,
erhält man beispielsweise, indem man Verbindungen der Formel (VI) 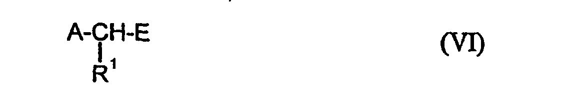 in welcher
- A und R¹: die oben angegebenen Bedeutungen haben und
- E: für eine Abgangsgruppe wie z.B. CI steht,
mit Verbindungen der Formel (VII) 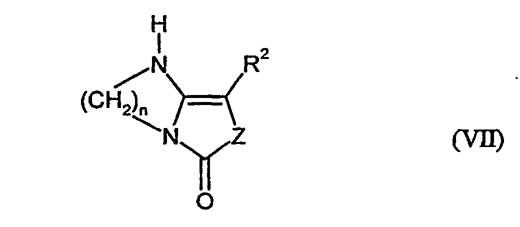 in welcher
- R², n und Z: die oben angegebenen Bedeutungen haben,
in Gegenwart eines Verdünnungsmittels wie z.B. N,N-Dimethylformamid, N,N-Dimethylacetamid, N-Methylpyyrrolidon oder Acetonitril und in Gegenwart von Säureakzeptoren bei Temperaturen zwischen 0°C und 200°C, vorzugsweise bei Temperaturen zwischen 20°C und 150°C umsetzt.

Als Säureakzeptoren können bei dem erfindungsgemäßen Verfahren alle üblicherweise für derartige Umsetzungen verwendbare Säurebindemittel eingesetzt werden.

Vorzugsweise in Frage kommen Alkalimetall- und Erdalkalimetall-hydride, wie Lithium-, Natrium-, Kalium- und Calcium-hydrid, Alkalimetall- und Erdalkalimetallhydroxide, wie Lithium-, Natrium-, Kalium- und Calcium-hydroxid, Alkalimetallund Erdalkalimetall-carbonate und hydrogencarbonate, wie Natrium- und Kaliumcarbonat oder Hydrogencarbonat sowie Calciumcarbonat, Alkalimetallacetate, wie Natrium- und Kalium-acetat, Alkalimetallalkoholate, wie Natrium- und Kaliummethylat, -ethylat, -propylat, -isopropylat, -butylat, -isobutylat und tert-butylat, ferner basische Stickstoffverbindungen, wie Trimethylamin, Triethylamin, Tripopylamin, Tributylamin, Düsobutylamin, Dicyclohexylamin, Ethyldiisopropylamin, Ethyldicyclohexylamin, Ethyldiisopropylamin, Ethyldicyclohexylamin, N,N-Dimethylbenzylamin, N,N-Dimethyl-analin, Pyridin, 2-Methyl-, 3-Methyl-, 4-Methyl, 2,4-Dimethyl-, 2,6-Dimethyl-, 2-Ethyl-, 4-Ethyl- und 5-Ethyl-2-Methyl-pyridin, 1,5-Diazabicyclo-[4,3,0]-non-5-en (DBN), 1,8-Diazabicyclo-[5,4,0]-undec-7-en (DBU) und 1,4-Diazabicyclo-[2,2,2]-octan (DABCO).

Die Verbindungen der Formel (VII) mit Z=S lassen sich beispielsweise aus Verbindungen der Formel (VIII) (vgl. R.C.F. Jones, J.R Nichols, Tetrahedron Lett. 31(12), 1771-1774,1990) auf folgendem Weg herstellen (vgl. US-Pat. 3,555,039). 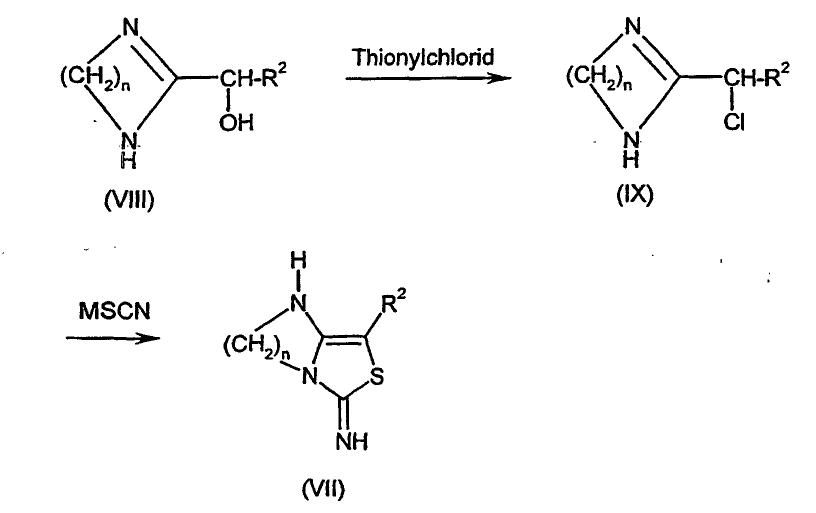 worin
- R²: die oben angegebene Bedeutung hat und
- M: für ein monovalentes Kation, wie beispielsweise Na oder K, steht.

Man erhält Verbindungen der Formel (IIIa) 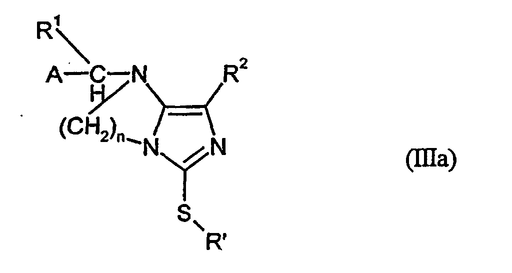 in welcher
- A, R¹ und R²: die oben angegebenen Bedeutungen haben und
- R': für Alkyl, bevorzugt Methyl oder Ethyl steht
beispielsweise, indem man Verbindungen der Formel (IVa) 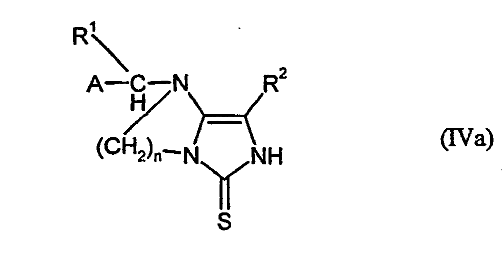 in welcher
- A, R¹ und R²: die oben angegebene Bedeutung haben,
zunächst mit einem Alkylhalogenid, bevorzugt einem Alkyliodid, insbesondere Methyliodid oder Ethyliodid, in Gegenwart eines Verdünnungsmittels wie Aceton umsetzt und dann mit einer Base wie Natriumcarbonat in Gegenwart eines Verdünnungsmittels wie Aceton das Endprodukt der Formel (IIIa) freisetzt.

Die Verbindungen der Formel (III) lassen sich auch direkt in solche Verbindungen der Formel (I) überführen, in welchen Y für CN steht. Geeignete Methoden sind z.B. angegeben in JP 7126483 und Arch. Pharm. 303(8), 625-633 (1970), deren Inhalt ausdrücklich Bestandteil dieser Anmeldung sein soll.

Verbindungen der Formel (I) lassen sich auch gemäß folgenden Reaktionsschema darstellen (vgl. die Herstellungsbeispiele):

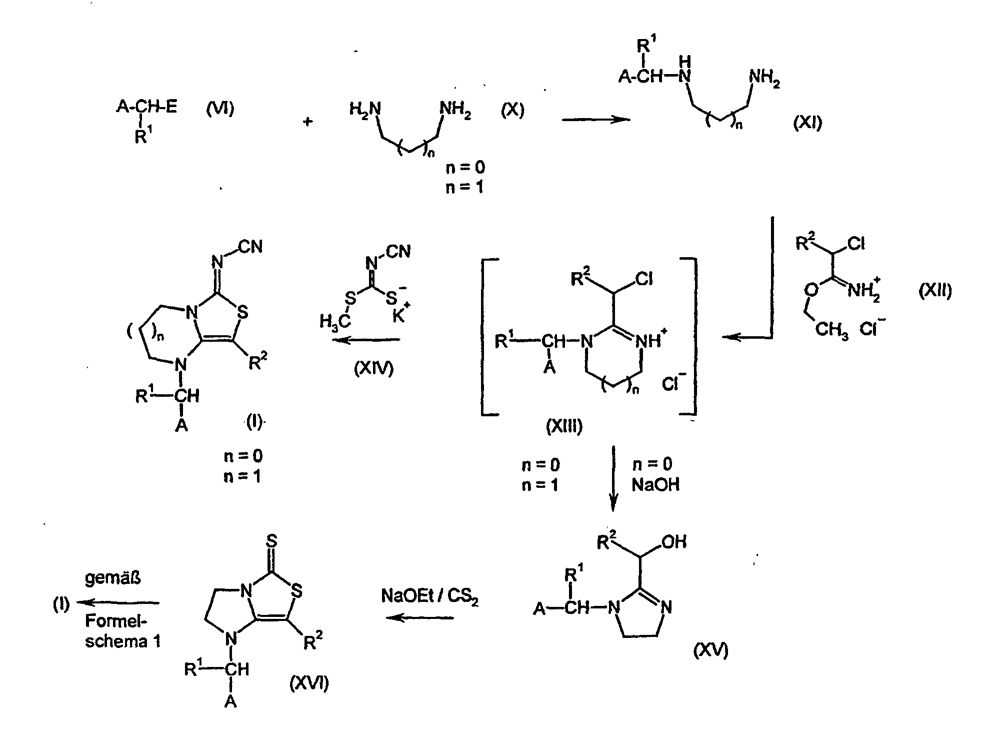

Die Verbindungen der Formel (I), in welchen Z für SO oder SO₂ steht, lassen sich aus Verbindungen der Formel (I), in welchen Z für S steht, durch Oxidation, nach üblichen Verfahren, beispielsweise mit geeigneten Oxidationsmitteln, wie Peroxide z. B. Wasserstoffperoxid, tert.-Butylperoxid, organische und anorganische Peroxide oder deren Salze wie 3-Chlorperbenzoesäure, Peressigsäure, Perameisensäure, Dibenzoylperoxid, Permangat, oder mit einem Gemisch aus Kaliumperoxomonosulfat, 2 KHSO₅, KHSO₄ und einem Lösungsmittel oder Lösungsmittelgemisch (z.B. Wasser, Essigsäure, Methanol, Methylenchlorid) erhalten. Man kann das Peroxid auch in-situ aus einem anderen Peroxid, beispielsweise Peressigsäure aus Essigsäure und Wasserstoffperoxid herstellen (vgl. auch A. R Katritzky, C.W. Rees in Comprehensive Heterocyclic Chemistry, Pergamon Press, Oxford, New York, 1984, Vol. 3, S. 96; O.J. Brown et al. Chem. Soc. (C), 1971, S. 256).

Die Oxidation kann auch mittels geeigneter Katalysatoren gestartet oder beschleunigt werden.

Die Wirkstoffe eignen sich bei guter Pflanzenverträglichkeit und günstiger Warmblütertoxizität zur Bekämpfung von tierischen Schädlingen, insbesondere Insekten, Spinnentieren und Nematoden, die in der Landwirtschaft, in Forsten, im Vorrats- und Materialschutz sowie auf dem Hygienesektor vorkommen. Sie können vorzugsweise als Pflanzenschutzmittel eingesetzt werden. Sie sind gegen normal sensible und resistente Arten sowie gegen alle oder einzelne Entwicklungsstadien wirksam. Zu den oben erwähnten Schädlingen gehören:
Aus der Ordnung der Isopoda z.B. Oniscus asellus, Armadillidium vulgare, Porcellio scaber.
Aus der Ordnung der Diplopoda z.B. Blaniulus guttulatus.
Aus der Ordnung der Chilopoda z.B. Geophilus carpophagus, Scutigera spp..
Aus der Ordnung der Symphyla z.B. Scutigerella immaculata.
Aus der Ordnung der Thysanura z.B. Lepisma saccharina.
Aus der Ordnung der Collembola z.B. Onychiurus armatus.
Aus der Ordnung der Orthoptera z.B. Acheta domesticus, Gryllotalpa spp., Locusta migratoria migratorioides, Melanoplus spp., Schistocerca gregaria.
Aus der Ordnung der Blattaria z.B. Blatta orientalis, Periplaneta americana, Leucophaea maderae, Blattella germanica.
Aus der Ordnung der Dermaptera z.B. Forficula auricularia.
Aus der Ordnung der Isoptera z.B. Reticulitermes spp..
Aus der Ordnung der Phthiraptera z.B. Pediculus humanus corporis, Haematopinus spp., Linognathus spp., Trichodectes spp., Damalinia spp..
Aus der Ordnung der Thysanoptera z.B. Hercinothrips femoralis, Thrips tabaci, Thrips palmi, Frankliniella accidentalis.
Aus der Ordnung der Heteroptera z.B. Eurygaster spp., Dysdercus intermedius, Piesma quadrata, Cimex lectularius, Rhodnius prolixus, Triatoma spp.
Aus der Ordnung der Homoptera z.B. Aleurodes brassicae, Bemisia tabaci, Trialeurodes vaporariorum, Aphis gossypii, Brevicoryne brassicae, Cryptomyzus ribis, Aphis fabae, Aphis pomi, Eriosoma lanigerum, Hyalopterus arundinis, Phylloxera vastatrix, Pemphigus spp., Macrosiphum avenae, Myzus spp., Phorodon humuli, Rhopalosiphum padi, Empoasca spp., Euscelis bilobatus, Nephotettix cincticeps, Lecanium corni, Saissetia oleae, Laodelphax striatellus, Nilaparvata lugens, Aonidiella aurantii, Aspidiotus hederae, Pseudococcus spp., Psylla spp.
Aus der Ordnung der Lepidoptera z.B. Pectinophora gossypiella, Bupalus piniarius, Cheimatobia brumata, Lithocolletis blancardella, Hyponomeuta padella, Plutella xylostella, Malacosoma neustria, Euproctis chrysorrhoea, Lymantria spp., Bucculatrix thurberiella, Phyllocnistis citrella, Agrotis spp., Euxoa spp., Feltia spp., Earias insulana, Heliothis spp., Mamestra brassicae, Panolis flammea, Spodoptera spp., Trichoplusia ni, Carpocapsa pomonella, Pieris spp., Chilo spp., Pyrausta nubilalis, Ephestia kuehniella, Galleria mellonella, Tineola bisselliella, Tinea pellionella, Hofmannophila pseudospretella, Cacoecia podana, Capua reticulana, Choristoneura fumiferana, Clysia ambiguella, Homona magnanima, Tortrix viridana, Cnaphalocerus spp., Oulema oryzae.
Aus der Ordnung der Coleoptera z.B. Anobium punctatum, Rhizopertha dominica, Bruchidius obtectus, Acanthoscelides obtectus, Hylotrupes bajulus, Agelastica alni, Leptinotarsa decemlineata, Phaedon cochleariae, Diabrotica spp., Psylliodes chrysocephala, Epilachna varivestis, Atomaria spp., Oryzaephilus surinamensis, Anthonomus spp., Sitophilus spp., Otiorrhynchus sulcatus, Cosmopolites sordidus, Ceuthorrhynchus assimilis, Hypera postica, Dermestes spp., Trogoderma spp., Anthrenus spp., Attagenus spp., Lyctus spp., Meligethes aeneus, Ptinus spp., Niptus hololeucus, Gibbium psylloides, Tribolium spp., Tenebrio molitor, Agriotes spp., Conoderus spp., Melolontha melolontha, Amphimallon solstitialis, Costelytra zealandica, Lissorhoptrus oryzophilus.
Aus der Ordnung der Hymenoptera z.B. Diprion spp., Hoplocampa spp., Lasius spp., Monomorium pharaonis, Vespa spp.
Aus der Ordnung der Diptera z.B. Aedes spp., Anopheles spp., Culex spp., Drosophila melanogaster, Musca spp., Fannia spp., Calliphora erythrocephala, Lucilia spp., Chrysomyia spp., Cuterebra spp., Gastrophilus spp., Hyppobosca spp., Stomoxys spp., Oestrus spp., Hypoderma spp., Tabanus spp., Tannia spp., Bibio hortulanus, Oscinella frit, Phorbia spp., Pegomyia hyoscyami, Ceratitis capitata, Dacus oleae, Tipula paludosa, Hylemyia spp., Liriomyza spp..
Aus der Ordnung der Siphonaptera z.B. Xenopsylla cheopis, Ceratophyllus spp..
Aus der Klasse der Arachnida z.B. Scorpio maurus, Latrodectus mactans, Acarus siro, Argas spp., Ornithodoros spp., Dermanyssus gallinae, Eriophyes ribis, Phyllocoptruta oleivora, Boophilus spp., Rhipicephalus spp., Amblyomma spp., Hyalomma spp., Ixodes spp., Psoroptes spp., Chorioptes spp., Sarcoptes spp., Tarsonemus spp., Bryobia praetiosa, Panonychus spp., Tetranychus spp., Hemitarsonemus spp., Brevipalpus spp..

Zu den pflanzenparasitären Nematoden gehören z.B. Pratylenchus spp., Radopholus similis, Ditylenchus dipsaci, Tylenchulus semipenetrans, Heterodera spp., Globodera spp., Meloidogyne spp., Aphelenchoides spp., Longidorus spp., Xiphinema spp., Trichodorus spp., Bursaphelenchus spp..

Die erfindungsgemäßen Verbindungen der Formel (I) zeichnen sich insbesondere durch Wirkung gegen saugende Insekten aus.

Die erfindungsgemäßen Verbindungen können gegebenenfalls in bestimmten Konzentrationen bzw. Aufwandmengen auch als Herbizide und Mikrobizide, beispielsweise als Fungizide, Antimykotika und Bakterizide verwendet werden. Sie lassen sich gegebenenfalls auch als Zwischen- oder Vorprodukte für die Synthese weiterer Wirkstoffe einsetzen.

Erfindungsgemäß können alle Pflanzen und Pflanzenteile behandelt werden. Unter Pflanzen werden hierbei alle Pflanzen und Pflanzenpopulationen verstanden, wie erwünschte und unerwünschte Wildpflanzen oder Kulturpflanzen (einschließlich natürlich vorkommender Kulturpflanzen). Kulturpflanzen können Pflanzen sein, die durch konventionelle Züchtungs- und Optimierungsmethoden oder durch biotechnologische und gentechnologische Methoden oder Kombinationen dieser Methoden erhalten werden können, einschließlich der transgenen Pflanzen und einschließlich der durch Sortenschutzrechte schützbaren oder nicht schützbaren Pflanzensorten. Unter Pflanzenteilen sollen alle oberirdischen und unterirdischen Teile und Organe der Pflanzen, wie Sproß, Blatt, Blüte und Wurzel verstanden werden, wobei beispielhaft Blätter, Nadeln, Stengel, Stämme, Blüten, Fruchtkörper, Früchte und Samen sowie Wurzeln, Knollen und Rhizome aufgeführt werden. Zu den Pflanzenteilen gehört auch Emtegut sowie vegetatives und generatives Vermehrungsmaterial, beispielsweise Stecklinge, Knollen, Rhizome, Ableger und Samen.

Die erfindungsgemäße Behandlung der Pflanzen und Pflanzenteile mit den Wirkstoffen erfolgt direkt oder durch Einwirkung auf deren Umgebung, Lebensraum oder Lagerraum nach den üblichen Behandlungsmethoden, z.B. durch Tauchen, Sprühen, Verdampfen, Vernebeln, Streuen, Aufstreichen und bei Vermehrungsmaterial, insbesondere bei Samen, weiterhin durch ein- oder mehrschichtiges Umhüllen.

Die Wirkstoffe können in die üblichen Formulierungen überführt werden, wie Lösungen, Emulsionen, Spritzpulver, Suspensionen, Pulver, Stäubemittel, Pasten, lösliche Pulver, Granulate, Suspemsions-Emulsions-Konzentrate, Wirkstoff-imprägnierte Natur- und synthetische Stoffe sowie Feinstverkapselungen in polymeren Stoffen.

Diese Formulierungen werden in bekannter Weise hergestellt, z.B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage:
z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnussschalen, Maiskolben und Tabakstengeln; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylaryl-polyglykolether, Alkylsulfonate, Alkylsulfate, Arylsulfonate sowie Einweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gew.-% Wirkstoff, vorzugsweise zwischen 0,5 und 90 %.

Der erfindungsgemäße Wirkstoff kann in seinen handelsüblichen Formulierungen sowie in den aus diesen Formulierungen bereiteten Anwendungsformen in Mischung mit anderen Wirkstoffen, wie Insektiziden, Lockstoffen, Sterilantien, Bakteriziden, Akariziden, Nematiziden, Fungiziden, wachstumsregulierenden Stoffen oder Herbiziden vorliegen. Zu den Insektiziden zählen beispielsweise Phosphorsäureester, Carbamate, Carbonsäureester, chlorierte Kohlenwasserstoffe, Phenylharnstoffe, durch Mikroorganismen hergestellte Stoffe u.a.

Besonders günstige Mischpartner sind z.B. die folgenden:
**Fungizide:**
   Aldimorph, Ampropylfos, Ampropylfos-Kalium, Andoprim, Anilazin, Azaconazol, Azoxystrobin,
   Benalaxyl, Benodanil, Benomyl, Benzamacril, Benzamacryl-isobutyl, Bialaphos, Binapacryl, Biphenyl, Bitertanol, Blasticidin-S, Bromuconazol, Bupirimat, Buthiobat, Calciumpolysulfid, Capsimycin, Captafol, Captan, Carbendazim, Carboxin, Carvon, Chinomethionat (Quinomethionat), Chlobenthiazon, Chlorfenazol, Chloroneb, Chloropicrin, Chlorothalonil, Chlozolinat, Clozylacon, Cufraneb, Cymoxanil, Cyproconazol, Cyprodinil, Cyprofuram, Debacarb, Dichlorophen, Diclobutrazol, Diclofluanid, Diclomezin, Dicloran, Diethofencarb, Difenoconazol, Dimethirimol, Dimethomorph, Diniconazol, Diniconazol-M, Dinocap, Diphenylamin, Dipyrithione, Ditalnmfos, Dithianon, Dodemorph, Dodine, Drazoxolon,
   Ediphenphos, Epoxiconazol, Etaconazol, Ethirimol, Etridiazol,
   Famoxadon, Fenapanil, Fenarimol, Fenbuconazol, Fenfuram, Fenitropan, Fenpiclonil, Fenpropidin, Fenpropimorph, Fentinacetat, Fentinhydroxyd, Ferbam, Ferimzon, Fluazinam, Flumetover, Fluoromid, Fluquinconazol, Flurprimidol, Flusilazol, Flusulfamid, Flutolanil, Flutriafol, Folpet, Fosetyl-Alminium, Fosetyl-Natcium, Fthalid, Fuberidazol, Furalaxyl, Furametpyr, Furcarbonil, Furconazol, Furconazol-cis, Furmecyclox,
   Guazatin,
   Hexachlorobenzol, Hexaconazol, Hymexazol,
   Imazalil, Imibenconazol, Iminoctadin, Iminoctadinealbesilat, Iminoctadinetriacetat, Iodocarb, Ipconazol, Iprobenfos (IBP), Iprodione, Irumamycin, Isoprothiolan, Isovaledione,
   Kasugamycin, Kresoxim-methyl, Kupfer-Zubereitungen, wie: Kupferhydroxid, Kupfernaphthenat, Kupferoxychlorid, Kupfersulfat, Kupferoxid, Oxin-Kupfer und Bordeaux-Mischung,
   Mancopper, Mancozeb, Maneb, Meferimzone, Mepanipyrim, Mepronil, Metalaxyl, Metconazol, Methasulfocarb, Methfuroxam, Metiram, Metomeclam, Metsulfovax, Mildiomycin, Myclobutanil, Myclozolin,
   Nickel-dimethyldithiocarbamat, Nitrothal-isopropyl, Nuarimol,
   Ofurace, Oxadixyl, Oxamocarb, Oxolinicacid, Oxycarboxim, Oxyfenthiin,
   Paclobutrazol, Pefurazoat, Penconazol, Pencycuron, Phosdiphen, Pimaricin, Piperalin, Polyoxin, Polyoxorim, Probenazol, Prochloraz, Procymidon, Propamocarb, Propanosine-Natrium, Propiconazol, Propineb, Pyrazophos, Pyrifenox, Pyrimethanil, Pyroquilon, Pyroxyfur,
   Quinconazol, Quintozen (PCNB),
   Schwefel und Schwefel-Zubereitungen,
   Tebuconazol, Tecloftalam, Tecnazen, Tetcyclacis, Tetraconazol, Thiabendazol, Thicyofen, Thifluzamide, Thiophanate-methyl, Thiram, Tioxymid, Tolclofos-methyl, Tolylfluanid, Triadimefon, Triadimenol, Triazbutil, Triazoxid, Trichlamid, Tricyclazol, Tridemorph, Triflumizol, Triforin, Triticonazol,
   Uniconazol,
   Validamycin A, Vinclozolin, Viniconazol,
   Zarilamid, Zineb, Ziram sowie
   Dagger G,
   OK-8705,
   OK-8801,
   α-(1,1-Dimethylethyl)-β-(2-phenoxyethyl)-1H-1,2,4-triazol-1-ethanol,
   α-(2,4-Dichlorphenyl)-β-fluor-b-propyl-1H-1,2,4-triazol-1-ethanol,
   α-(2,4-Dichlorphenyl)-β-methoxy-a-methyl-1H-1,2,4-triazol-1-ethanol,
   α-(5-Methyl-1,3-dioxan-5-yl)-β-[[4-(trifluormethyl)-phenyl]-methylen]-1H-1,2,4-triazol-1-ethanol,
   (5RS,6RS)-6-Hydroxy-2,2,7,7-tetramethyl-5-(1H-1,2,4-triazol-1-yl)-3-octanon,
   (E)-a-(Methoxyimino)-N-methyl-2-phenoxy-phenylacetamid,
   {2-Methyl-1-[[[1-(4-methylphenyl)-ethyl]-amino]-carbonyl]-propyl}-carbaminsäure-1-isopropylester
   1-(2,4-Dichlorphenyl)-2-(1H-1,2,4-triazol-1-yl)-ethanon-O-(phenylmethyl)-oxim, 1-(2-Methyl-1-naphthalenyl)-1H-pyrrol-2,5-dion,
   1-(3,5-Dichlorphenyl)-3-(2-propenyl)-2,5-pyrrolidinndion,
   1-[(Diiodmethyl)-sulfonyl]-4-methyl-benzol,
   1-[[2-(2,4-Dichlorphenyl)-1,3-dioxolan-2-yl]-methyl]-1H-imidazol,
   1-[[2-(4-Chlorphenyl)-3-phenyloxiranyl]-methyl]-1H-1,2,4-triazol,
   1-[1-[2-[(2,4-Dichlorphenyl)-methoxyl-phenyl]-ethenyl]-1H-imidazol,
   1-Methyl-5-nonyl-2-(phenylmethyl)-3-pyrrolidinol,
   2',6'-Dibrom-2-methyl-4'-trifluormethoxy-4'-trifluor-methyl-1,3-thiazol-5-carboxanilid,
   2,2-Dichlor-N-[1-(4-chlorphenyl)-ethyl]-1-ethyl-3-methyl-cyclopropancarboxamid,
   2,6-Dichlor-5-(methylthio)-4-pyrimidinyl-thiocyanat,
   2,6-Dichlor-N-(4-trifluormethylbenzyl)-benzamid,
   2,6-Dichlor-N-[[4-(trifluormethyl)-phenyl]-methyl]-benzamid,
   2-(2,3,3-Triiod-2-propenyl)-2H-tetrazol,
   2-[(1-Methylethyl)-sulfonyl]-5-(trichlormethyl)-1,3,4-thiadiazol,
   2-[[6-Deoxy-4-O-(4-O-methyl-β-D-glycopyranosyl)-a-D-glucopyranosyl]-amino]-4-methoxy-1H-pyrrolo[2,3-d]pyrimidin-5-carbonitril,
   2-Aminobutan,
   2-Brom-2-(brommethyl)-pentandinitril,
   2-Chlor-N-(2,3-dihydro-1,1,3-trimethyl-1H-inden-4-yl)-3-pyridincarboxamid,
   2-Chlor-N-(2,6-dimethylphenyl)-N-(isothiocyanatomethyl)-acetamid,
   2-Phenylphenol(OPP),
   3,4-Dichlor-1-[4-(difluormethoxy)-phenyl]-1H-pyrrol-2,5-dion,
   3,5-Dichlor-N-[cyan[(1-methyl-2-propynyl)-oxy]-methyl]-benzamid,
   3-(1,1-Dimethylpropyl-1-oxo-1H-inden-2-carbonitril,
   3-[2-(4-Chlorphenyl)-5-ethoxy-3-isoxazolidinyl]-pyridin,
   4-Chlor-2-cyan-N,N-dimethyl-5-(4-methylphenyl)-1H-imidazol-1-sulfonamid,
   4-Methyl-tetrazolo[1,5-a]quinazolin-5(4H)-on,
   8-(1,1-Dimethylethyl)-N-ethyl-N-propyl-1,4-dioxaspiro[4.5]decan-2-methanamin,
   8-Hydroxychinolinsulfat,
   9H-Xanthen-9-carbonsäure-2-[(phenylamino)-carbonyl]-hydrazid,
   bis-(1-Methylethyl)-3-methyl-4-[(3-methylbenzoyl)-oxy]-2,5-thiophendicarboxylat,
   cis-1-(4-Chlorphenyl)-2-(1H-1,2,4-triazol-1-yl)-cycloheptanol,
   cis-4-[3-[4-(1,1-Dimethylpropyl)-phenyl-2-methylpropyl]-2,6-dimethyl-morpholinhydrochlorid,
   Ethyl-[(4-chlorphenyl)-azo]-cyanoacetat,
   Kaliumhydrogencarbonat,
   Methantetrathiol-Natriumsalz,
   Methyl-1-(2,3-dihydro-2,2-dimethyl-1H-inden-1-yl)-1H-imidazol-5-carboxylat,
   Methyl-N-(2,6-dimethylphenyl)-N-(5-isoxazolylcarbonyl)-DL-alaninat,
   Methyl-N-(chloracetyl)-N-(2,6-dimethylphenyl)-DL-alaninat,
   N-(2,3-Dichlor-4-hydroxyphenyl)-1-methyl-cyclohexancarboxamid.
   N-(2,6-Dimethylphenyl)-2-methoxy-N-(tetrahydro-2-oxo-3-furanyl)-acetamid,
   N-(2,6-Dimethylphenyl)-2-methoxy-N-(tetrahydro-2-oxo-3-thienyl)-acetamid,
   N-(2-Chlor-4-nitrophenyl)-4-methyl-3-nitro-benzolsulfonamid,
   N-(4-Cyclohexylphenyl)-1,4,5,6-tetrahydro-2-pyrimidinamin,
   N-(4-Hexylphenyl)-1,4,5,6-tetrahydro-2-pyrimidinamin,
   N-(5-Chlor-2-methylphenyl)-2-methoxy-N-(2-oxo-3-oxazolidinyl)-acetamid,
   N-(6-Methoxy)-3-pyridinyl)-cyclopropancarboxamid,
   N-[2,2,2-Trichlor-1-[(chloracetyl)-amino]-ethyl]-benzamid,
   N-[3-Chlor-4,5-bis-(2-propinyloxy)-phenyl]-N'-methoxy-methanimidamid,
   N-Formyl-N-hydroxy-DL-alanin -Natriumsalz,
   O,O-Diethyl-[2-(dipropylamino)-2-oxoethyl]-ethylphosphoramidothioat,
   O-Methyl-S-phenyl-phenylpropylphosphoramidothioate,
   S-Methyl-1,2,3-benzothiadiazol-7-carbothioat,
   spiro[2H]-1-Benzopyran-2,1'(3'H)-isobenzofuran]-3'-on,
**Bakterizide:**
   Bronopol, Dichlorophen, Nitrapyrin, Nickel-Dimethyldithiocarbamat, Kasugamycin, Octhilinon, Furancarbonsäure, Oxytetracyclin, Probenazol, Streptomycin, Tecloftalam, Kupfersulfat und andere Kupfer-Zubereitungen.
**Insektizide / Akarizide / Nematizide:**
   Abamectin, Acephate, Acetamiprid, Acrinathrin, Alanycarb, Aldicarb, Aldoxycarb, Alpha-cypermethrin, Alphamethrin, Amitraz, Avermectin, AZ 60541, Azadirachtin, Azamethiphos, Azinphos A, Azinphos M, Azocyclotin,
   Bacillus popilliae, Bacillus sphaericus, Bacillus subtilis, Bacillus thuringiensis, Baculoviren, Beauveria bassiana, Beauveria tenella, Bendiocarb, Benfuracarb, Bensultap, Benzoximate, Betacyfluthrin, Bifenazate, Bifenthrin, Bioethanomethrin, Biopermethrin, BPMC, Bromophos A, Bufencarb, Buprofezin, Butathiofos, Butocarboxim, Butylpyridaben,
   Cadusafos, Carbaryl, Carbofuran, Carbophenothion, Carbosulfan, Cartap, Chloethocarb, Chlorethoxyfos, Chlorfenapyr, Chlorfenvinphos, Chlorfluazuron, Chlormephos, Chlorpyrifos, Chlorpyrifos M, Chlovaporthrin, Cis-Resmethrin, Cispermethrin, Clocythrin, Cloethocarb, Clofentezine, Cyanophos, Cycloprene, Cycloprothrin, Cyfluthrin, Cyhalothrin, Cyhexatin, Cypermethrin, Cyromazine,
   Deltamethrin, Demeton M, Demeton S, Demeton-S-methyl, Diafenthiuron, Diazinon, Dichlorvos, Diflubenzuron, Dimethoat, Dimethylvinphos, Diofenolan, Disulfoton, Docusat-sodium, Dofenapyn,
   Eflusilanate, Emamectin, Empenthrin, Endosulfan, Entomopfthora spp., Eprinomectin, Esfenvalerate, Ethiofencarb, Ethion, Ethoprophos, Etofenprox, Etoxazole, Etrimfos,
   Fenamiphos, Fenazaquin, Fenbutatin oxide, Fenitrothion, Fenothiocarb, Fenoxacrim, Fenoxycarb, Fenpropathrin, Fenpyrad, Fenpyrithrin, Fenpyroximate, Fenvalerate, Fipronil, Fluazinam, Fluazuron, Flubrocythrinate, Flucycloxuron, Flucythrinate, Flufenoxuron, Flutenzine, Fluvalinate, Fonophos, Fosmethilan, Fosthiazate, Fubfenprox, Furathiocarb,
   Granuloseviren
   Halofenozide, HCH, Heptenophos, Hexaflumuron, Hexythiazox, Hydroprene,
   Imidacloprid, Isazofos, Isofenphos, Isoxathion, Ivennectin,
   Kernpolyederviren
   Lambda-cyhalothrin, Lufenuron
   Malathion, Mecarbam, Metaldehyd, Methamidophos, Metharhizium anisopliae, Metharhizium flavoviride, Methidathion, Methiocarb, Methomyl, Methoxyfenozide, Metolcarb, Metoxadiazone, Mevinphos, Milbemectin, Monocrotophos,
   Naled, Nitenpyram, Nithiazine, Novaluron
   Omethoat, Oxamyl, Oxydemethon M
   Paecilomyces fumosoroseus, Parathion A, Parathion M, Permethrin, Phenthoat, Phorat, Phosalone, Phosmet, Phosphamidon, Phoxim, Pirimicarb, Pirimiphos A, Pirimiphos M, Profenofos, Promecarb, Propoxur, Prothiofos, Prothoat, Pymetrozine, Pyraclofos, Pyresmethrin, Pyrethrum, Pyridaben, Pyridathion, Pyrimidifen, Pyriproxyfen,
   Quinalphos,
   Ribavirin
   Salithion, Sebufos, Selamectin, Silafluofen, Spinosad, Sulfotep, Sulprofos,
   Tau-fluvalinate, Tebufenozide, Tebufenpyrad, Tebupirimiphos, Teflubenzuron,
   Tefluthrin, Temephos, Temivinphos, Terbufos, Tetrachlorvinphos, Thetacypermethrin, Thiamethoxam, Thiapronil, Thiatriphos, Thiocyclam hydrogen oxalate, Thiodicarb, Thiofanox, Thuringiensin, Tralocythrin, Tralomethrin, Triarathene, Triazamate, Triazophos, Triazuron, Trichlophenidine, Trichlorfon, Triflumuron, Trimethacarb,
   Vamidothion, Vaniliprole, Verticillium lecanii
   YI 5302
   Zeta-cypermethrin, Zolaprofos
   (1R-cis)-[5-(Phenylmethyl)-3-furanyl]-methyl-3-[(dihydro-2-oxo-3(2H)-furanyliden)-methyl]-2,2-dimethylcyclopropancarboxylat
   (3-Phenoxyphenyl)-methyl-2,2,3,3-tetramethylcyclopropanecarboxylat 1-[(2-Chlor-5-thiazolyl)methyl]tetrahydro-3,5-dimethyl-N-nitro-1,3,5-triazin-2(1H)-imin
   2-(2-Chlor-6-fluorphenyl)-4-[4-.(1,1-dimethylethyl)phenyl]-4,5-dihydro-oxazol
   2-(Acetlyoxy)-3-dodecyl-1,4-naphthalindion
   2-Chlor-N-[[[4-(1-phenylethoxy)-phenyl]-amino]-carbonyl]-benzamid 2-Chlor-N-[[[4-(2,2-dichlor-1,1-difluorethoxy)-phenyl]-amino]-carbonyl]-benzamid 3-Methylphenyl-propylcarbamat
   4-[4-(4-Ethoxyphenyl)-4-methylpentyl]-1-fluor-2-phenoxy-benzol
   4-Chlor-2-(1,1-dimethylethyl)-5-[[2-(2,6-dimethyl-4-phenoxyphenoxy)ethyl]thio]-3(2H)-pyridazinon
   4-Chlor-2-(2-chlor-2-methylpropyl)-5-[(6-iod-3-pyridinyl)methoxy]-3(2H)-pyridazinon
   4-Chlor-5-[(6-chlor-3-pyridinyl)methoxy]-2-(3,4-dichlorphenyl)-3(2H)-pyridazinon Bacillus thuringiensis strain EG-2348
   Benzoesäure [2-benzoyl-1-(1,1-dimethylethyl)-hydrazid
   Butansäure 2,2-dimethyl-3-(2,4-dichlorphenyl)-2-oxo-1-oxaspiro[4.5]dec-3-en-4-ylester
   [3-[(6-Chlor-3-pyridinyl)methyl]-2-thiazolidinyliden]-cyanamid
   Dihydro-2-(nitromethylen)-2H-1,3-thiazine-3(4H)-carboxaldehyd
   Ethyl-[2-[[1,6-dihydro-6-oxo-1-(phenylmethyl)-4-pyridazinyl]oxy]ethyl]-carbamat
   N-(3,4,4-Trifluor-1-oxo-3-butenyl)-glycin
   N-(4-Chlorphenyl)-3-[4-(difluormethoxy)phenyl]-4,5-dihydro-4-phenyl-1H-pyrazol-1-carboxamid
   N-[(2-Chlor-5-thiazolyl)methyl]-N'-methyl-N"-nitro-guanidin
   N-Methyl-N'-(1-methyl-2-propenyl)-1,2-hydrazindicarbothioamid
   N-Methyl-N'-2-propenyl-1,2-hydrazindicarbothioamid
   O,O-Diethyl-[2-(dipropylamino)-2-oxoethyl]-ethylphosphoramidothioat

Auch eine Mischung mit anderen bekannten Wirkstoffen, wie Herbiziden oder mit Düngemitteln und Wachstumsregulatoren ist möglich.

Die erfindungsgemäßen Wirkstoffe können ferner beim Einsatz als Insektizide in ihren handelsüblichen Formulierungen sowie in den aus diesen Formulierungen bereiteten Anwendungsformen in Mischung mit Synergisten vorliegen. Synergisten sind Verbindungen, durch die die Wirkung der Wirkstoffe gesteigert wird, ohne dass der zugesetzte Synergist selbst aktiv wirksam sein muss.

Der Wirkstoffgehalt der aus den handelsüblichen Formulierungen bereiteten Anwendungsformen kann in weiten Bereichen variieren. Die Wirkstoffkonzentration der Anwendungsformen kann von 0,0000001 bis zu 95 Gew.-% Wirkstoff, vorzugsweise zwischen 0,0001 und 1 Gew.-% liegen.

Die Anwendung geschieht in einer den Anwendungsformen angepassten üblichen Weise.

Bei der Anwendung gegen Hygiene- und Vorratsschädlinge zeichnet sich der Wirkstoff durch eine hervorragende Residualwirkung auf Holz und Ton sowie durch eine gute Alkalistabilität auf gekälkrten Unterlagen aus.

Die erfindungsgemäßen Wirkstoffe wirken nicht nur gegen Pflanzen-, Hygiene- und Vorratsschädlinge, sondern auch auf dem veterinärmedizinischen Sektor gegen tierische Parasiten (Ektoparasiten) wie Schildzecken, Lederzecken, Räudemilben, Laufmilben, Fliegen (stechend und leckend), parasitierende Fliegenlarven, Läuse, Haarlinge, Federlinge und Flöhe. Zu diesen Parasiten gehören:
Aus der Ordnung der Anoplurida z.B. Haematopinus spp., Linognathus spp., Pediculus spp., Phtirus spp., Solenopotes spp..
Aus der Ordnung der Mallophagida und den Unterordnungen Amblycerina sowie Ischnocerina z.B. Trimenopon spp., Menopon spp., Trinoton spp., Bovicola spp., Werneckiella spp., Lepikentron spp., Damalina spp., Trichodectes spp., Felicola spp..
Aus der Ordnung Diptera und den Unterordnungen Nematocerina sowie Brachycerina z.B. Aedes spp., Anopheles spp., Culex spp., Simulium spp., Eusimulium spp., Phlebotomus spp., Lutzomyia spp., Culicoides spp., Chrysops spp., Hybomitra spp., Atylotus spp., Tabanus spp., Haematopota spp., Philipomyia spp., Braula spp., Musca spp., Hydrotaea spp., Stomoxys spp., Haematobia spp., Morellia spp., Fannia spp., Glossina spp., Calliphora spp., Lucilia spp., Chrysomyia spp., Wohlfahrtia spp., Sarcophaga spp., Oestrus spp., Hypoderma spp., Gasterophilus spp., Hippobosca spp., Lipoptena spp., Melophagus spp..
Aus der Ordnung der Siphonapterida z.B. Pulex spp., Ctenocephalides spp., Xenopsylla spp., Ceratophyllus spp..
Aus der Ordnung der Heteropterida z.B. Cimex spp., Triatoma spp., Rhodnius spp., Panstrongylus spp..
Aus der Ordnung der Blattarida z.B. Blatta orientalis, Periplaneta americana, Blattela germanica, Supella spp..
Aus der Unterklasse der Acaria (Acarida) und den Ordnungen der Meta- sowie Mesostigmata z.B. Argas spp., Ornithodorus spp., Otobius spp., Ixodes spp., Amblyomma spp., Boophilus spp., Dermacentor spp., Haemophysalis spp., Hyalomma spp., Rhipicephalus spp., Dermanyssus spp., Raillietia spp., Pneumonyssus spp., Stemostoma spp., Varroa spp..
Aus der Ordnung der Actinedida (Prostigmata) und Acaridida (Astigmata) z.B. Acarapis spp., Cheyletiella spp., Ornithocheyletia spp., Myobia spp., Psorergates spp., Demodex spp., Trombicula spp., Listrophorus spp., Acarus spp., Tyrophagus spp., Caloglyphus spp., Hypodectes spp., Pterolichus spp., Psoroptes spp., Chorioptes spp., Otodectes spp., Sarcoptes spp., Notoedres spp., Knemidocoptes spp., Cytodites spp., Laminosioptes spp..

Beispielsweise zeigen sie eine gute Wirksamkeit gegen Aphis spp. und Myzus spp.

Die erfindungsgemäßen Wirkstoffe der Formel (I) eignen sich auch-zur Bekämpfung von Arthropoden, die landwirtschaftliche Nutztiere, wie z.B. Rinder, Schafe, Ziegen, Pferde, Schweine, Esel, Kamele, Büffel, Kaninchen, Hühner, Puten, Enten, Gänse, Bienen, sonstige Haustiere wie z.B. Hunde, Katzen, Stubenvögel, Aquarienfische sowie sogenannte Versuchstiere, wie z.B. Hamster, Meerschweinchen, Ratten und Mäuse befallen. Durch die Bekämpfung dieser Arthropoden sollen Todesfälle und Leistungsminderungen (bei Fleisch, Milch, Wolle, Häuten, Eiern, Honig usw.) vermindert werden, so dass durch den Einsatz der erfindungsgemäßen Wirkstoffe eine wirtschaftlichere und einfachere Tierhaltung möglich ist.

Die Anwendung der erfindungsgemäßen Wirkstoffe geschieht im Veterinärsektor in bekannter Weise durch enterale Verabreichung in Form von beispielsweise Tabletten, Kapseln, Tränken, Drenchen, Granulaten, Pasten, Boli, des feed-through-Verfahrens, von Zäpfchen, durch parenterale Verabreichung, wie zum Beispiel durch Injektionen (intramuskulär, subcutan, intravenös, intraperitonal u.a.), Implantate, durch nasale Applikation, durch dermale Anwendung in Form beispielsweise des Tauchens oder Badens (Dippen), Sprühens (Spray), Aufgießens (Pour-on und Spot-on), des Waschens, des Einpuderns sowie mit Hilfe von wirkstoffhaltigen Formkörpern, wie Halsbändern, Ohrmarken, Schwanzmarken, Gliedmaßenbändern, Halftern, Markierungsvorrichtungen usw.

Bei der Anwendung für Vieh, Geflügel, Haustiere etc. kann man die Wirkstoffe der Formel (I) als Formulierungen (beispielsweise Pulver, Emulsionen, fließfähige Mittel), die die Wirkstoffe in einer Menge von 1 bis 80 Gew.-% enthalten, direkt oder nach 100 bis 10 000-facher Verdünnung anwenden oder sie als chemisches Bad verwenden.

Außerdem wurde gefunden, dass die erfindungsgemäßen Verbindungen eine hohe insektizide Wirkung gegen Insekten zeigen, die technische Materialien zerstören.

Beispielhaft und vorzugsweise - ohne jedoch zu limitieren - seien die folgenden Insekten genannt:
Käfer wie
   Hylotrupes bajulus, Chlorophorus pilosis, Anobium punctatum, Xestobium rufovillosum, Ptilinus pecticornis, Dendrobium pertinex, Emobius mollis, Priobium carpini, Lyctus brunneus, Lyctus africanus, Lyctus planicollis, Lyctus Iinearis, Lyctus pubescens, Trogoxylon aequale, Minthes rugicollis, Xyleborus spec. Tryptodendron spec. Apate monachus, Bostrychus capucins, Heterobostrychus brunneus, Sinoxylon spec. Dinoderus minutus.
Hautflügler wie
   Sirexjuvencus, Urocerus gigas, Urocerus gigas taignus, Urocerus augur.
Termiten wie
   Kalotermes flavicollis, Cryptotermes brevis, Heterotennes indicola, Reticulitermes flavipes, Reticulitermes santonensis, Reticulitermes lucifugus, Mastotermes darwiniensis, Zootermopsis nevadensis, Coptotermes formosanus.

Borstenschwänze wie Lepisma saccharina.

Unter technischen Materialien sind im vorliegenden Zusammenhang nicht-lebende Materialien zu verstehen, wie vorzugsweise Kunststoffe, Klebstoffe, Leime, Papiere und Kartone, Leder, Holz, Holzverarbeitungsprodukte und Anstrichmittel.

Ganz besonders bevorzugt handelt es sich bei dem vor Insektenbefall zu schützenden Material um Holz und Holzverarbeitungsprodukte.

Unter Holz und Holzverarbeitungsprodukten, welche durch das erfindungsgemäße Mittel bzw. dieses enthaltende Mischungen geschützt werden kann; ist beispielhaft zu verstehen:
Bauholz, Holzbalken, Eisenbahnschwellen, Brückenteile, Bootsstege, Holzfahrzeuge, Kisten, Paletten, Container, Telefonmasten, Holzverkleidungen, Holzfenster und - türen, Sperrholz, Spanplatten, Tischlerarbeiten oder Holzprodukte, die ganz allgemein beim Hausbau oder in der Bautischlerei Verwendung finden.

Die Wirkstoffe können als solche, in Form von Konzentraten oder allgemein üblichen Formulierungen wie Pulver, Granulate, Lösungen, Suspensionen, Emulsionen oder Pasten angewendet werden.

Die genannten Formulierungen können in an sich bekannter Weise hergestellt werden, z.B. durch Vermischen der Wirkstoffe mit mindestens einem Lösungs- bzw. Verdünnungsmittel, Emulgator, Dispergier- und/oder Binde- oder Fixiermittels, Wasser-Repellent, gegebenenfalls Sikkative und UV-Stabilisatoren und gegebenenfalls Farbstoffen und Pigmenten sowie weiteren Verarbeitungshilfsmitteln.

Die zum Schutz von Holz und Holzwerkstoffen verwendeten insektiziden Mittel oder Konzentrate enthalten den erfindungsgemäßen Wirkstoff in einer Konzentration von 0,0001 bis 95 Gew.-%, insbesondere 0,001 bis 60 Gew.-%.

Die Menge der eingesetzten Mittel bzw. Konzentrate ist von der Art und dem Vorkommen der Insekten und von dem Medium abhängig. Die optimale Einsatzmenge kann bei der Anwendung jeweils durch Testreihen ermittelt werden. Im allgemeinen ist es jedoch ausreichend 0,0001 bis 20 Gew.-%, vorzugsweise 0,001 bis 10 Gew.-%, des Wirkstoffs, bezogen auf das zu schützende Material, einzusetzen.

Als Lösungs- und/oder Verdünnungsmittel dient ein organisch-chemisches Lösungsmittel oder Lösungsmittelgemisch und/oder ein öliges oder ölartiges schwer flüchtiges organisch-chemisches Lösungsmittel oder Lösungsmittelgemisch und/oder ein polares organisch-chemisches Lösungsmittel oder Lösungsmittelgemisch und/oder Wasser und gegebenenfalls einen Emulgator und/oder Netzmittel.

Als organisch-chemische Lösungsmittel werden vorzugsweise ölige oder ölartige Lösungsmittel mit einer Verdunstungszahl über 35 und einem Flammpunkt oberhalb 30°C, vorzugsweise oberhalb 45°C, eingesetzt. Als derartige schwerflüchtige, wasserunlösliche, ölige und ölartige Lösungsmittel werden entsprechende Mineralöle oder deren Aromatenfraktionen oder mineralölhaltige Lösungsmittelgemische, vorzugsweise Testbenzin, Petroleum und/oder Alkylbenzol verwendet

Vorteilhaft gelangen Mineralöle mit einem Siedebereich von 170 bis 220°C, Testbenzin mit einem Siedebereich von 170 bis 220°C, Spindelöl mit einem Siedebereich von 250 bis 350°C, Petroleum bzw. Aromaten vom Siedebereich von 160 bis 280°C, Terpentinöl und dgl. zum Einsatz.

In einer bevorzugten Ausführungsform werden flüssige aliphatische Kohlenwasserstoffe mit einem Siedebereich von 180 bis 210°C oder hochsiedende Gemische von aromatischen und aliphatischen Kohlenwasserstoffen mit einem Siedebereich von 180 bis 220°C und/oder Spindeöl und/oder Monochlornaphthalin, vorzugsweise α-Monochlornaphthalin, verwendet

Die organischen schwerflüchtigen öligen oder ölartigen Lösungsmittel mit einer Verdunstungszahl über 35 und einem Flammpunkt oberhalb 30°C, vorzugsweise oberhalb 45°C, können teilweise durch leicht oder mittelflüchtige organisch-chemische Lösungsmittel ersetzt werden, mit der Maßgabe, dass das Lösungsmittelgemisch ebenfalls eine Verdunstungszahl über 35 und einen Flammpunkt oberhalb 30°C, vorzugsweise oberhalb 45°C, aufweist und dass das Insektizid-Fungizid-Gemisch in diesem Lösungsmittelgemisch löslich oder emulgierbar ist.

Nach einer bevorzugten Ausführungsform wird ein Teil des organisch-chemischen Lösungsmittel oder Lösungsmittelgemisches oder ein aliphatisches polares organisch-chemisches Lösungsmittel oder Lösungsmittelgemisch ersetzt. Vorzugsweise gelangen Hydroxyl- und/oder Ester- und/oder Ethergruppen enthaltende aliphatische organisch-chemische Lösungsmittel wie beispielsweise Glycolether, Ester oder dgl. zur Anwendung.

Als organisch-chemische Bindemittel werden im Rahmen der vorliegenden Erfindung die an sich bekannten wasserverdünnbaren und/oder in den eingesetzten organisch-chemischen Lösungsmitteln löslichen oder dispergier- bzw. emulgierbaren Kunstharze und/oder bindende trocknende Öle, insbesondere Bindemittel bestehend aus oder enthaltend ein Acrylatharz, ein Vinylharz, z.B. Polyvinylacetat, Polyesterharz, Polykondensations- oder Polyadditionsharz, Polyurethanharz, Alkydharz bzw. modifiziertes Alkydharz, Phenolharz, Kohlenwasserstoffharz wie Inden-Cumaronharz, Siliconharz, trocknende pflanzliche und/oder trocknende Öle und/oder physikalisch trocknende Bindemittel auf der Basis eines Natur- und/oder Kunstharzes verwendet

Das als Bindemittel verwendete Kunstharz kann in Form einer Emulsion, Dispersion oder Lösung, eingesetzt werden. Als Bindemittel können auch Bitumen oder bituminöse Substanzen bis zu 10 Gew.-%, verwendet werden. Zusätzlich können an sich bekannte Farbstoffe, Pigmente, wasserabweisende Mittel, Geruchskorrigentien und Inhibitoren bzw. Korrosionsschutzmittel und dgl. eingesetzt werden.

Bevorzugt ist gemäß der Erfindung als organisch-chemische Bindemittel mindestens ein Alkydharz bzw. modifiziertes Alkydharz und/oder ein trocknendes pflanzliches Öl im Mittel oder im Konzentrat enthalten. Bevorzugt werden gemäß der Erfindung Alkydharze mit einem Ölgehalt von mehr als 45 Gew.-%, vorzugsweise 50 bis 68 Gew.-%, verwendet.

Das erwähnte Bindemittel kann ganz oder teilweise durch ein Fixierungsmittel(gemisch) oder ein Weichmacher(gemisch) ersetzt werden. Diese Zusätze sollen einer Verflüchtigung der Wirkstoffe sowie einer Kristallisation bzw. Ausfällen vorbeugen. Vorzugsweise ersetzen sie 0,01 bis 30 % des Bindemittels (bezogen auf 100 % des eingesetzten Bindemittels).

Die Weichmacher stammen aus den chemischen Klassen der Phthalsäureester wie Dibutyl-, Dioctyl- oder Benzylbutylphthalat, Phosphorsäureester wie Tributylphosphat, Adipinsäureester wie Di-(2-ethylhexyl)-adipat, Stearate wie Butylstearat oder Amylstearat, Oleate wie Butyloleat, Glycerinether oder höhermolekulare Glykolether, Glycerinester sowie p-Toluolsulfonsäureester.

Fixierungsmittel basieren chemisch auf Polyvinylalkylethem wie z.B. Polyvinylmethylether oder Ketonen wie Benzophenon, Ethylenbenzophenon.

Als Lösungs- bzw. Verdünnungsmittel kommt insbesondere auch Wasser in Frage, gegebenenfalls in Mischung mit einem oder mehreren der oben genannten organisch-chemischen Lösungs- bzw. Verdünnungsmittel, Emulgatoren und Dispergatoren.

Ein besonders effektiver Holzschutz wird durch großtechnische Imprägnierverfahren, z.B. Vakuum, Doppelvakuum oder Druckverfahren, erzielt.

Die anwendungsfertigen Mittel können gegebenenfalls noch weitere Insektizide und gegebenenfalls noch ein oder mehrere Fungizide enthalten.

Als zusätzliche Zumischpartner kommen vorzugsweise die in der WO 94/29 268 genannten Insektizide und Fungizide in Frage. Die in diesem Dokument genannten Verbindungen sind ausdrücklicher Bestandteil der vorliegenden Anmeldung.

Als ganz besonders bevorzugte Zumischpartner können Insektizide, wie Chlorpyriphos, Phoxim, Silafluofin, Alphamethrin, Cyfluthrin, Cypermethrin, Deltamethrin, Permethrin, Imidacloprid, NI-25, Flufenoxuron, Hexaflumuron, Transfluthrin, Thiacloprid, Methoxyphenoxid und Triflumuron,
sowie Fungizide wie Epoxyconazole, Hexaconazole, Azaconazole, Propiconazole, Tebuconazole, Cyproconazole, Metconazole, Imazalil, Dichlorfluanid, Tolylfluanid, 3-Iod-2-propinyl-butylcarbamat, N-Octyl-isothiazolin-3-on und 4,5-Dichlor-N-octylisothiazolin-3-on, sein.

Zugleich können die erfindungsgemäßen Verbindungen zum Schutz vor Bewuchs von Gegenständen, insbesondere von Schiffskörpern, Sieben, Netzen, Bauwerken, Kaianlagen und Signalanlagen, welche mit See- oder Brackwasser in Verbindung kommen, eingesetzt werden.

Bewuchs durch sessile Oligochaeten, wie Kalkröhrenwürmer sowie durch Muscheln und Arten der Gruppe Ledamorpha (Entenmuscheln), wie verschiedene Lepas- und Scalpellum-Arten, oder durch Arten der Gruppe Balanomorpha (Seepocken), wie Balanus- oder Pollicipes-Species, erhöht den Reibungswiderstand von Schiffen und führt in der Folge durch erhöhten Energieverbrauch und darüber hinaus durch häufige Trockendockaufenthalte zu einer deutlichen Steigerung der Betriebskosten.

Neben dem Bewuchs durch Algen, beispielsweise Ectocarpus sp. und Ceramium sp., kommt insbesondere dem Bewuchs durch sessile Entomostraken-Gruppen, welche unter dem Namen Cirripedia (Rankenflusskrebse) zusammengefasst werden, besondere Bedeutung zu.

Es wurde nun überraschenderweise gefunden, dass die erfindungsgemäßen Verbindungen allein oder in Kombination mit anderen Wirkstoffen, eine hervorragende Antifouling (Antibewuchs)-Wirkung aufweisen.

Durch Einsatz von erfindungsgemäßen Verbindungen allein oder in Kombination mit anderen Wirkstoffen, kann auf den Einsatz von Schwermetallen wie z.B. in Bis(trialkylzinn)-sulfiden, Tri-*n*-butylzinnlaurat, Tri-*n*-butylzinnchlorid, Kupfer(I)-oxid, Triethylzinnchlorid, Tri-*n*-butyl(2-phenyl-4-chlorphenoxy)-zinn, Tributylzinnoxid, Molybdändisulfid, Antimonoxid, polymerem Butyltitanat, Phenyl-(bispyridin)-wismutchlorid, Tri-*n*-butylzinnfluorid, Manganethylenbisthiocarbamat, Zinkdimethyldithiocarbamat, Zinkethylenbisthiocarbamat, Zink- und Kupfersalze von 2-Pyridinthiol-1-oxid, Bisdimethyldithiocarbamoylzinkethylenbisthiocarbamat, Zinkoxid, Kupfer(I)-ethylen-bisdithiocarbamat, Kupferthiocyanat, Kupfernaphthenat und Tributylzinnhalogeniden verzichtet werden oder die Konzentration dieser Verbindungen entscheidend reduziert werden.

Die anwendungsfertigen Antifoulingfarben können gegebenenfalls noch andere Wirkstoffe, vorzugsweise Algizide, Fungizide, Herbizide, Molluskizide bzw. andere Antifouling-Wirkstoffe enthalten.

Als Kombinationspartner für die erfindungsgemäßen Antifouling-Mittel eignen sich vorzugsweise:
Algizide wie
   2-*tert*.-Butylamino-4-cyclopropylamino-6-methylthio-1,3,5-triazin, Dichlorophen, Diuron, Endothal, Fentinacetat, Isoproturon, Methabenzthiazuron, Oxyfluorfen, Quinoclamine und Terbutryn;
Fungizide wie
   Benzo[*b*]thiophencarbonsäurecyclohexylamid-S,S-dioxid, Dichlofluanid, Fluorfolpet, 3-Iod-2-propinyl-butylcarbamat, Tolylfluanid und Azole wie Azaconazole, Cyproconazole, Epoxyconazole, Hexaconazole, Metconazole, Propiconazole und Tebuconazole;
Molluskizide wie
   Fentinacetat, Metaldehyd, Methiocarb, Niclosamid, Thiodicarb und Trimethacarb; oder herkömmliche Antifouling-Wirkstoffe wie 4,5-Dichlor-2-octyl-4-isothiazolin-3-on, Diiodmethylparatrylsulfon, 2-(N,N-Dimethylthiocarbamoylthio)-5-nitrothiazyl, Kalium-, Kupfer-, Natrium- und Zinksalze von 2-Pyridinthiol-l-oxid, Pyridin-triphenylboran, Tetrabutyldistannoxan, 2,3,5,6-Tetrachlor-4-(methylsulfonyl)-pyridin, 2,4,5,6-Tetrachloroisophthalonitril, Tetramethylthiuramdisulfid und 2,4,6-Trichlorphenylmaleinimid.

Die verwendeten Antifouling-Mittel enthalten die erfindungsgemäßen Wirkstoff der erfindungsgemäßen Verbindungen in einer Konzentration von 0,001 bis 50 Gew.-%, insbesondere von 0,01 bis 20 Gew.-%.

Die erfindungsgemäßen Antifouling-Mittel enthalten des weiteren die üblichen Bestandteile wie z.B. in Ungerer, *Chem. Ind.* **1985,** *37*, 730-732 und Williams, Antifouling Marine Coatings, Noyes, Park Ridge, **1973** beschrieben.

Antifouling-Anstrichmittel enthalten neben den algiziden, fungiziden, molluskiziden und erfindungsgemäßen insektiziden Wirkstoffen insbesondere Bindemittel.

Beispiele für anerkannte Bindemittel sind Polyvinylchlorid in einem Lösungsmittelsystem, chlorierter Kautschuk in einem Lösungsmittelsystem, Acrylharze in einem Lösungsmittelsystem insbesondere in einem wässrigen System, Vinylchlorid/Vinylacetat-Copolymersysteme in Form wässriger Dispersionen oder in Form von organischen Lösungsmittelsystemen, Butadien/Styrol/Acrylnitril-Kautschuke, trocknende Öle, wie Leinsamenöl, Harzester oder modifizierte Hartharze in Kombination mit Teer oder Bitumina, Asphalt sowie Epoxyverbindungen, geringe Mengen Chlorkautschuk, chloriertes Polypropylen und Vinylharze.

Gegebenenfalls enthalten Anstrichmittel auch anorganische Pigmente, organische Pigmente oder Farbstoffe, welche vorzugsweise in Seewasser unlöslich sind. Ferner können Anstrichmittel Materialien, wie Kolophonium enthalten, um eine gesteuerte Freisetzung der Wirkstoffe zu ermöglichen. Die Anstriche können ferner Weichmacher, die rheologischen Eigenschaften beeinflussende Modifizierungsmittel sowie andere herkömmliche Bestandteile enthalten. Auch in Self-Polishing-Antifouling-Systemen können die erfindungsgemäßen Verbindungen oder die oben genannten Mischungen eingearbeitet werden.

Die Wirkstoffe eignen sich auch zur Bekämpfung von tierischen Schädlingen, insbesondere von Insekten, Spinnentieren und Milben, die in geschlossenen Räumen, wie beispielsweise Wohnungen, Fabrikhallen, Büros, Fahrzeugkabinen u.ä. vorkommen. Sie können zur Bekämpfung dieser Schädlinge allein oder in Kombination mit anderen Wirk- und Hilfsstoffen in Haushaltsinsektizid-Produkten verwendet werden. Sie sind gegen sensible und resistente Arten sowie gegen alle Entwicklungsstadien wirksam. Zu diesen Schädlingen gehören:
Aus der Ordnung der Scorpionidea z.B. Buthus occitanus.
Aus der Ordnung der Acarina z.B. Argas persicus, Argas reflexus, Bryobia ssp., Dermanyssus gallinae, Glyciphagus domesticus, Ornithodorus moubat, Rhipicephalus sanguineus, Trombicula alfreddugesi, Neutrombicula autumnalis, Dermatophagoides pteronissimus, Dermatophagoides forinae.
Aus der Ordnung der Araneae z.B. Aviculariidae, Araneidae.
Aus der Ordnung der Opiliones z.B. Pseudoscorpiones chelifer, Pseudoscorpiones cheiridium, Opiliones phalangium.
Aus der Ordnung der Isopoda z.B. Oniscus asellus, Porcellio scaber.
Aus der Ordnung der Diplopoda z.B. Blaniulus guttulatus, Polydesmus spp..
Aus der Ordnung der Chilopoda z.B. Geophilus spp..
Aus der Ordnung der Zygentoma z.B. Ctenolepisma spp., Lepisma saccharina, Lepismodes inquilinus.
Aus der Ordnung der Blattaria z.B. Blatta orientalies, Blattella germanica, Blattella asahinai, Leucophaea maderae, Panchlora spp., Parcoblatta spp., Periplaneta australasiae, Periplaneta americana, Periplaneta brunnea, Periplaneta fuliginosa, Supella longipalpa.
Aus der Ordnung der Saltatoria z.B. Acheta domesticus.
Aus der Ordnung der Dermaptera z.B. Forficula auricularia.
Aus der Ordnung der Isoptera z.B. Kalotennes spp., Reticulitermes spp.
Aus der Ordnung der Psocoptera z.B. Lepinatus spp., Liposcelis spp.
Aus der Ordnung der Coleptera z.B. Anthrenus spp., Attagenus spp., Dermestes spp., Latheticus oryzae, Necrobia spp., Ptinus spp., Rhizopertha dominica, Sitophilus granarius, Sitophilus oryzae, Sitophilus zeamais, Stegobium paniceum.
Aus der Ordnung der Diptera z.B. Aedes aegypti, Aedes albopictus, Aedes taeniorhynchus, Anopheles spp., Calliphora erythrocephala, Chrysozona pluvialis, Culex quinquefasciatus, Culex pipiens, Culex tarsalis, Drosophila spp., Fannia canicularis, Musca domestica, Phlebotomus spp., Sarcophaga carnaria, Simulium spp., Stomoxys calcitrans, Tipula paludosa.
Aus der Ordnung der Lepidoptera z.B. Achroia grisella, Galleria mellonella, Plodia interpunctella, Tinea cloacella, Tinea pellionella, Tineola bisselliella.
Aus der Ordnung der Siphonaptera z.B. Ctenocephalides canis, Ctenocephalides felis, Pulex irritans, Tunga penetrans, Xenopsylla cheopis.
Aus der Ordnung der Hymenoptera z.B. Camponotus herculeanus, Lasius fuliginosus, Lasius niger, Lasius umbratus, Monomorium pharaonis, Paravespula spp., Tetramorium caespitum.
Aus der Ordnung der Anoplura z.B. Pediculus humanus capitis, Pediculus humanus corporis, Phthirus pubis.
Aus der Ordnung der Heteroptera z.B. Cimex hemipterus, Cimex lectularius, Rhodinus prolixus, Triatoma infestans.

Die Anwendung im Bereich der Haushaltsinsektizide erfolgt allein oder in Kombination mit anderen geeigneten Wirkstoffen wie Phosphorsäureestern, Carbamaten, Pyrethroiden, Wachstumsregulatoren oder Wirkstoffen aus anderen bekannten Insektizidklassen.

Die Anwendung erfolgt in Aerosolen, drucklosen Sprühmitteln, z.B. Pump- und Zerstäubersprays, Nebelautomaten, Foggern, Schäumen, Gelen, Verdampferprodukten mit Verdampferplättchen aus Cellulose oder Kunststoff, Flüssigverdampfern, Gel- und Membranverdampfern, propellergetriebenen Verdampfern, energielosen bzw. passiven Verdampfungssystemen, Mottenpapieren, Mottensäckchen und Mottengelen, als Granulate oder Stäube, in Streuködem oder Köderstationen.

### Herstellungsbeispiele

### Beispiel I-1

### 1-[6-Chlor-3-pyridylmethyl]-5-cyanimino-7-methyl-4,5-dihydro-[1,3]-diazolano-[1,2-c][1,3]-thiazolin

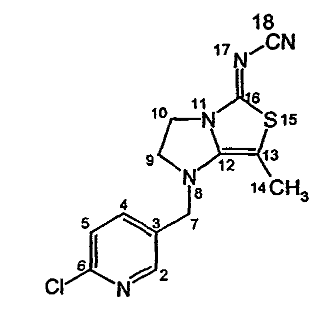

In einem 100 ml Zweihalskolben werden 10.0 mmol N-(6-Chlor-3-pyridylmethyl)-ethan-1,2-diamin (XI-1) in 25 ml abs. Ethanol vorgelegt und bei Raumtemperatur 1.89 g (11.0 mmol) 2-Chlorpropanethylesteriminiumchlorid (XII) in 20 ml abs. Ethanol zugetropft. Nach beendeter Zugabe wird eine Stunde bei Raumtemperatur gerührt. Anschließend werden 1.70 g (10.0 mmol) des Kalium-*N*-cyaniminomethylsulfanylinethanthiolat (XIV) portionsweise unter kräftigem Rühren zugegeben, eine Stunde bei Raumtemperatur gerührt und 90 Minuten unter Rückfluss erhitzt. Das Reaktionsgemisch wird heiß - direkt in 60 ml Wasser - filtriert. Das Filtrat wird über Nacht im Kühlschrank aufbewahrt, wobei die Produkte kristallisieren oder als Öl anfallen. In beiden Fällen wird der Niederschlag aus Ethanol umkristallisiert.
- Smp. :: 130°C
- Ausbeute :: 0.82 g (27 % d. Th.)
¹H - NMR ₃₀₀ (DMSO-d₆ / TMS)
δ / ppm = 2.04 (s, 3 H, H-14), 3.71 (m, 2 H, H-9), 3.87 (m, 2 H, H-10), 4.49 (s, 2 H, H-7), 7.56 (d, 1 H, H-5, *J =* 8.2 Hz), 7.87 (dd, 1 H, H-4, *J =* 8.2 Hz, *J =* 2.5 Hz), 8.43 (d, 1 H, H-2, *J* = 2.5 Hz).

### Beispiel I-2

### 1-[6-Chlor-3-pyridylmethyl]-6-cyanimino-8-methyl-1,2,3,4,5,8a,5,6-oktahydro-[1,3]-thiazolino-[3,4-a] pyrimidin

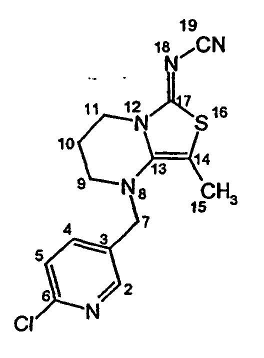

wird analog Beispiel I-1 unter Verwendung von N-(6-Chlor-3-pyridylmethyl)-propan-1,2-diamin (XI-2) hergestellt.
- Smp. :: 153°C
- Ausbeute :: 1.09 g (34 % d. Th.)
¹H - NMR ₃₀₀ (DMSO-d₆ / TMS)
δ / ppm = 1.92 (qui, 2 H, H-10, ^{*3*}*J =* 5.9 Hz), 2.06 (s, 3 H, H-15), 3.05 (t, 2 H, H-9, ^{*3*}*J* = 5.6 Hz), 3.77 (t, 2 H, H-11, ^{*3*}*J* = 6.3 Hz), 4.40 (s, 2 H, H-7), 7.55 (d, 1 H, H-5, *J =* 8.2 Hz), 7.88 (dd, 1 H, H-4, *J =* 8.2 Hz, *J* = 2.5 Hz), 8.41 (d, 1 H, H-2, *J =* 2.5 Hz).

### Beispiel (XI-1)

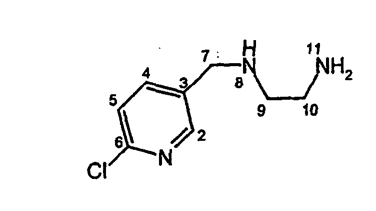

In 100 ml abs. Acetonitril werden 0.06 mol Ethan-1,2-diamin (3.61 g) bei Raumtemperatur mit 2.6 ml abs. Triethylamin versetzt. Innerhalb von zwei Stunden werden 3.24 g (0.02 mol) 6-Chlor-3-chlormethyl-pyridin (CCMP) in 100 ml abs. Acetonitril sehr langsam zugetropft und 20 Stunden gerührt. Hiernach wird das Reaktionsgemisch filtriert, unter vermindertem Druck am Rotationsverdampfer vom Lösungsmittel befreit und im Hochvakuum fraktioniert destilliert.
- Sdp. :: 120°C / 0.05 mbar
- Ausbeute :: 2.45 g (65 % d. Th.)
¹H - NMR ₃₀₀ (CDCl₃ / TMS)
δ / ppm = 1.41 (br, 3 H, H-8 u. H-11), 2.68 (m, 2 H, H-10), 2.83 (m, 2 H, H-9), 3.80 (s, 2 H, H-7), 7.29 (d, 1 H, H-5, *J* = 8.1 Hz), 7.68 (dd, 1 H, H-4, *J* = 8.1 Hz, *J =* 2.5 Hz), 8.34 (d, 1 H, H-2, J= 2.5 Hz).

### Beispiel (XI-2)

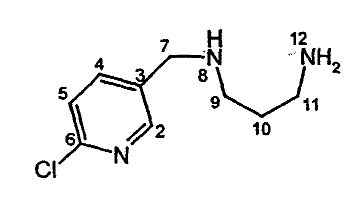

wird analog Beispiel (XI-1) unter Verwendung von Propan-1,2-diamin (4.45 g) erhalten.
- Sdp. :: 125°C / 0.05 mbar
- Ausbeute :: 2.48 g (62 % d. Th.)
¹H - NMR ₃₀₀ (CDCl₃ / TMS)
δ / ppm = 1.38 (br, 3 H, H-8 u. H-12), 1,65 (qui, 2 H, H-10, ^{*3*}*J* = 6.8 Hz), 2.69 (t, 2 H, H-11, ^{*3*}*J* = 6.8 Hz), 2.78 (t, 2 H, H-9, ^{*3*}*J* = 6.8 Hz), 3.79 (s, 2 H, H-7), 7.29 (d, 1 H, H-5, *J =* 8.1 Hz), 7.68 (dd, 1 H, H-4, *J =* 8.1 Hz, *J =* 2.4 Hz), 8.34 (d, 1 H, H-2, *J =* 2.4 Hz).

### Beispiel (XIV)

### Kalium-N-cyaniminomethylsulfanylmethanthiolat

### a) Herstellung des Dikaliumsalzes der Cyanimidodithiokohlensäure

In einem 500 ml Vierhalskolben mit KPG-Rührer, Tropftrichter, Thermometer und Rückflusskühler werden unter Stickstoffatmosphäre 21.0 g (0.50 mol) Cyanamid in 40 ml Ethanol auf 0°C gebracht. Bei dieser Temperatur werden nacheinander 41.9 g (0.55 mol) Schwefelkohlenstoff und dann 56.1 g (1.00 mol) Kaliumhydroxid in 180 ml Ethanol so zugetropft, dass die Innentemperatur nicht 5°C übersteigt (ca. vier Stunden). Nach beendeter Zugabe wird das Kühlbad entfernt und über Nacht bei Raumtemperatur gerührt. Der entstandene Feststoff wird filtriert (sehr feiner Niederschlag setzt schnell die Poren der Fritten zu), mit Ethanol gewaschen und im Vakuum getrocknet. Das entstandene Salz ist hygroskopisch und sollte nur kurze Zeit beim filtrieren mit Luft/Luftfeuchtigkeit in Berührung kommen. Das Salz wird im nächsten Schritt als Rohprodukt eingesetzt Die Ausbeute liegt bei 75-80 % der Theorie.

### b) Kalium-N-cyaniminomethylsulfanylmethanthiolat

77.7 g (0.40 mol) des Rohproduktes werden in einem Gemisch aus 270 ml Aceton und 300 ml Wasser gelöst und auf 0°C gebracht. Bei dieser Temperatur werden unter starkem Rühren 56.8 g (0.40 mol) Methyliodid in 150 ml Aceton langsam über drei Stunden zugetropft. Nach beendeter Zugabe wir das Reaktionsgemisch eine Stunde bei 0°C und drei Stunden. bei Raumtemperatur weitergerührt. Am Rotationsverdampfer wird bis zur Trockne alle Lösemittel entfernt. Der Rückstand wird in 250 ml abs. Aceton aufgenommen und bis auf die Hälft am Rotationsverdampfer eingeengt. Zur Kristallisation des entstandenen Kaliumiodids wird das Reaktionsgemisch über Nacht im Kühlschrank belassen. Der entstandene Feststoff wird filtriert und das Filtrat weiter fast bis zur Trockne eingeengt. In der Kälte fällt das gewünschte Produkt. Der letzte Vorgang zur vollständigen Entfernung des Kaliumiodids kann nun wiederholt werden. Die Ausbeute liegt bei 75-80 % der Theorie. Die analytische Daten stimmen mit der Literatur überein.

### Beispiel (XII-1)

### 2-Chlorpropanethylesteriminiumchlorid

### (vgl. S. Shibata et al., Bull. Chem. Soc. Jpn. 55(11), 3546-3551 (1982))

In einem mit Stickstoff sekurierten und tarierten Kolben werden 35.00 g abs. Diethylether, 2.30 g (50.0 mmol) abs. Ethanol und 4.48 g (50.0 mmol) 2-Chlorpropannitril auf 0 - 5°C gebracht. Sodann wird unter mäßigem Rühren innerhalb von 45 - 60 Minuten langsam 1.80 g (50.0 mmol) durch konz. Schwefelsäure getrocknetes Chlorwasserstoffgas eingeleitet. Die eingeleitete Menge an- Chlorwasserstoff wird durch Zurückwiegen des Kolbens bestimmt. Hierbei ist auf ständigen Feuchtigkeitsausschluss zu achten. Das Reaktionsgemisch wird unter ständigen Rühren innerhalb von 20 Stunden auf Raumtemperatur gebracht, wobei das Produkt als weißes Pulver kristallisiert. Zur vollständigen Kristallisation wird das Reaktionsgemisch einen weiteren Tag in der Kälte aufbewahrt, filtriert und das Rohprodukt, das ohne weitere Aufarbeitung verwendet wird, im Hochvakuum bei Raumtemperatur getrocknet. Da geringe Spuren an Wasser das Produkt zersetzen, sind keine analytischen Untersuchungen angestellt worden. Die Ausbeute liegt bei 50 - 60 % der Theorie.

### Beispiel (XV-1)

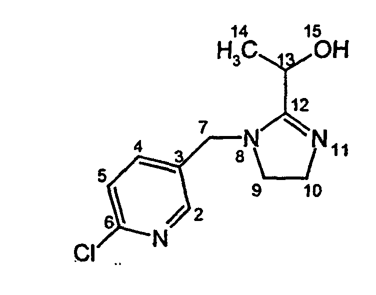

In 25 ml abs. Ethanol werden 1.86 g (10.0 mmol) *N*-(6-Chlor-3-pyridylmethyl)-ethan-1,2-diamin (XI-1) gelöst und bei Raumtemperatur mit einer Lösung aus 1.89 g (11.0 mmol) 2-Chlor-propanethylesteriminiumchlorid (XII-1) in 20 ml abs. Ethanol tropfenweise versetzt. Nach beendeter Zugabe wird eine Stunde bei Raumtemperatur gerührt. Zum Austreiben des entstandenen Ammoniaks wird unter kräftigem Rühren vier Stunden ein mäßiger Stickstoffstrom durch das Reaktionsgemisch geleitet, filtriert und unter vermindertem Druck das Lösungsmittel am Rotationsverdampfer entfernt. Der ölige Rückstand wird in 25 ml Ethanol aufgenommen, auf dem Eisbad mit 4.0 ml 10 % Natronlauge versetzt und eine Stunde bei Raumtemperatur gerührt. Zur Isolierung des Produktes wird filtriert, das Lösungsmittel entfernt und der ölige Rückstand mit Ethylacetat / Methanol 2 / 1 (R_{f}= 0.2) über Kieselgel eluiert.
- Ausbeute :: 1.69 g (71 % d. Th.)
¹H - NMR ₃₀₀ (DMSO-d₆ / TMS)
δ / ppm = 1.22 (d, 3 H, H-14, ^{*3*}*J* = 6.9 Hz), 2.83 (ddd, 1 H, H-9, ^{*2*}*J* = 12.8 Hz, ^{*3*}*J = 9.8* Hz, ^{*3*}*J =* 4.3 Hz), 2.99 (ddd, 1 H, H-9, ^{*2*}*J* = 12.8 Hz, ^{*3*}*J*= 4.7 Hz, ^{*3*}*J* = 3.3 Hz), 3.15 (dt, 1 H, H-10, ^{*2*}*J* = 11.4 Hz, ^{*3*}*J* = 4.0 Hz), 3.31 (ddd, 1 H, H-10, ^{*2*}*J =* 11.4 Hz, ^{*3*}*J* = 9.8 Hz, ^{*3*}*J* = 4.7 Hz), 4.51 (m, 2 H, H-7), 7.50 (d, 1 H, H-5, *J =* 8.2 Hz), 7.73 (dd, 1 H, H-4, *J =* 8.2 Hz, *J =* 2.5 Hz), 8.32 (d, 1 H, H-2, *J =* 2.5 Hz).

### Beispiel XVI-1)

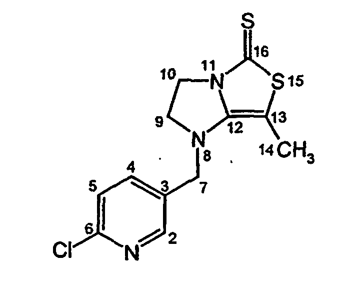

0.76 g (10.0 mmol) Schwefelkohlenstoff in 10 ml abs. Ethanol werden auf dem Eisbad mit einer Lösung aus 0.23 g (10.0 mmol) Natrium in 5 ml abs. Ethanol tropfenweise versetzt und nach beendeter Zugabe 30 Minuten bei Raumtemperatur gerührt. Diese Lösung wird auf dem Eisbad in das Rohprodukt (XV-1), das in 25 ml Ethanol gelöst ist, getropft, langsam auf Raumtemperatur gebracht und 90 Minuten unter Rückfluss erhitzt. Anschließend wird heiß filtriert und das Filtrat eingeengt. Das Produkt fällt in Form blass gelber Kristalle an. Zur Reinigung wird aus Ethanol umkristallisiert.
- Smp. :: 126°C
- Ausbeute :: 1.36 g (54 % d. Th. bezogen auf XV-1)
¹H - NMR ₃₀₀ (DMSO-d₆ / TMS)
δ / ppm = 2.01 (s, 3 H, H-14), 3.72 (m, 2 H, H-9), 3.88 (m, 2 H, H-10), 4.47 (s, 2 H, H-7), 7.55 (d, 1 H, H-5, *J =* 8.2 Hz), 7.88 (dd, 1 H, H-4, *J =* 8.2 Hz, *J* = 2.5 Hz), 8.44 (d, 1 H, H-2, *J* = 2.5 Hz).

### Anwendungsbeispiele

### Beispiel 1

### Test mit Schaben - Tauchverfahren

- Testtiere:: Dritte Larvenstadien von *Periplaneta americana*
- Lösungsmittel:: Dimethylsulfoxid

20 mg Wirkstoff werden in einem ml Dimethylsulfoxid gelöst. Zwecks Herstellung einer geeigneten Formulierung verdünnt man die Wirkstofflösung mit Wasser auf die jeweils gewünschte Konzentration.

20 ml dieser Wirkstoffzubereitung werden in Röhrchen (⌀ 1,5 cm, H 10 cm) pipettiert. 4 Schabenlarven werden mit CO₂ betäubt und ein Röhrchen (⌀ 1,2 cm, H 9 cm) mit 3 Löchern (Boden und 5 cm unter dem oberen Rand) überführt. Das Röhrchen wird mit einem Stopfen verschlossen und 30 min bei Raumtemperatur gehalten, bis alle Schabenlarven wieder normale Aktivität zeigen. Das Röhrchen wird 60 Sekunden in die Wirkstoffzubereitung getaucht wobei alle Schabenlarven vollständig benetzt werden. Nach Ablaufen der Flüssigkeit werden die Schabenlarven auf Filterscheiben in PP-Dosen (⌀ 9,7 cm, H 8 cm) überführt.

Nach 2 und 24 Stunden sowie nach 7 Tagen wird die Wirksamkeit der Wirkstoffzubereitung ermittelt. Dabei bedeutet 100 %, dass alle Schabenlarven abgetötet wurden; 0 % bedeutet, dass keine Schabenlarven abgetötet wurden.

Bei diesem Test zeigen z.B. die folgenden Verbindungen der Herstellungsbeispiele eine ausreichende Wirkung:

| | % Wirkung (7d) | | |
|---|---|---|---|
| Verbindung | 1000 ppm | 300 ppm | 100 ppm |
| I-1 | 100 | 100 | 100 |

### Beispiel 2

### Blowfly-Larven-Test / Entwicklungshemmende Wirkung

- Testtiere:: Lucilia cuprina-Larven
- Lösungsmittel:: Dimethylsulfoxid

20 mg Wirkstoff werden in einem ml Dimethylsulfoxid gelöst. Zwecks Herstellung einer geeigneten Formulierung verdünnt man die Wirkstofflösung mit Wasser auf die jeweils gewünschte Konzentration.

Etwa 20 Lucilia cuprina-Larven werden in ein Teströhrchen gebracht, welches ca. 1 cm³ Pferdefleisch und 0.5 ml der zu testende Wirkstoffzubereitung enthält. Nach 48 Stunden wird die Wirksamkeit der Wirkstoffzubereitung als % Larvenmortalität ermittelt.

Die Teströhrchen werden anschließend in Becher mit Sand-bedecktem Boden überführt. Nach weiteren 12 Tagen werden die Teströhrchen entfernt und die Puppen und Fliegen ausgezählt. Als entwicklungsinhibitorische Wirkung wird die Schlupfhemmung nach 1,5-facher Entwicklungsdauer einer unbehandelten Kontolle in % (Verhältnis von Puppen zu geschlüpften Fliegen) angegeben.

Bei diesem Test zeigen z.B. die folgenden Verbindungen der Herstellungsbeispiele eine ausreichende Wirkung:

| | % Wirkung (Larvenmortalität) | | |
|---|---|---|---|
| Verbindung | 1000 ppm | 300 ppm | 100 ppm |
| I-1 | 100 | 100 | 100 |

### Beispiel 3

### Test mit Katzenflöhen / orale Aufnahme

- Testtiere:: Adulte von *Ctenocephalides felis*
- Lösungsmittel:: Dimethylsulfoxid (DMSO)

Zwecks Herstellung einer geeigneten Formulierung wird aus 20 mg Wirkstoff mit 1 ml DMSO eine geeignete Wirkstofflösung hergestellt. 20 µl dieser Formulierung werden zu 4 ml citriertem Rinderblut gegeben und verrührt.

20 nüchterne adulte Flöhe *(Ctenocephalides felis,* Stamm "Georgi") werden in eine Kammer(⌀ 5 cm) eingesetzt, die oben und unten mit Gaze verschlossen ist. Auf die Kammer wird ein Metallzylinder gestellt, dessen Unterseite mit Parafilm verschlossen ist. Der Zylinder enthält die 4 ml Blut-Wirkstofformulierung, die von den Flöhen durch die Parafilmmembran aufgenommen werden kann. Während das Blut auf 37°C erwärmt wird, wird im Bereich der Flohkammern eine Temperatur von 25°C eingestellt. Kontrollen werden mit dem gleichen Volumen DMSO ohne Zusatz einer Verbindung vermischt. Es werden Dreifach-Bestimmungen durchgeführt.

Nach 24h wird die Mortalität in % bestimmt.

Verbindung die innerhalb von 24 h eine mindestens 25 %ige Abtötung der Flöhe erzielen werden als wirksam beurteilt.

Bei diesem Test zeigen z.B. die folgenden Verbindungen der Herstellungsbeispiele eine Wirkung:

| | % Wirkung (Larvenmortalität) |
|---|---|
| Verbindung | 100 ppm |
| I-1 | 30 |

## Patentansprüche

1. Verbindungen der Formel (I) 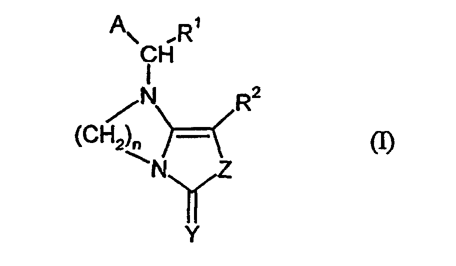 in welcher
A für jeweils gegebenenfalls substituiertes Aryl oder Hetaryl oder Heterocyclyl steht,
R¹ für Wasserstoff oder C₁-C₃-Alkyl steht,
R² für Wasserstoff, C₁-C₃-Alkyl oder jeweils gegebenenfalls substituiertes Aryl oder Hetaryl steht,
n x für 2, 3 oder 4 steht,
Y für N-CN oder N-NO₂ steht,
Z für S, SO, SO₂ oder NR³ steht und
R³ für Wasserstoff oder C₁-C₃-Alkyl steht.

2. Verbindungen der Formel (I) gemäß Anspruch 1, worin
A für gegebenenfalls durch Halogen, Cyano, Nitro, C₁-C₄-Alkyl, C₁-C₄-Halogenalkyl, C₁-C₄-Alkoxy oder C₁-C₄-Halogenalkoxy substituiertes Phenyl steht oder
A für Pyrazolyl, 1,2,4-Triazolyl, Oxazolyl, Isoxazolyl, Thiazolyl, Isothiazolyl, 1,2,5-Thiadiazolyl, Pyridyl, Pyrazinyl oder Pyrimidinyl steht, welche gegebenenfalls durch Fluor, Chlor, Brom, Cyano, Nitro, C₁-C₂-Alkyl (welches gegebenenfalls durch Fluor und/oder Chlor substituiert ist), C₁-C₂-Alkoxy (welches gegebenenfalls durch Fluor und/oder Chlor substituiert ist), C₁-C₂-Alkylthio (welches gegebenenfalls durch Fluor und/oder Chlor substituiert ist) oder C₁-C₂-Alkylsulfonyl (welches gegebenenfalls durch Fluor und/oder Chlor substituiert ist) substituiert sind, oder
A für einen gegebenenfalls durch Halogen oder C₁-C₃-Alkyl substituierten gesättigten C₅-C₆-Cycloalkylrest steht, in welchem eine Methylengruppe durch O oder S ersetzt ist,
R¹ für Wasserstoff, Methyl, Ethyl, n-Propyl oder i-Propyl steht,
R² für Wasserstoff, Methyl, Ethyl, n-Propyl, i-Propyl, für gegebenenfalls durch Halogen, Cyano, Nitro, C₁-C₄-Alkyl, C₁-C₄-Halogenalkyl, C₁-C₄-Alkoxy oder C₁-C₄-Halogenalkoxy substituiertes Phenyl oder für Pyrazolyl, 1,2,4-Triazolyl, Oxazolyl, Isoxazolyl, Thiazolyl, Isothiazolyl, 1,2,5-Thiadiazolyl, Pyridyl, Pyrazinyl oder Pyrimidinyl steht, welche gegebenenfalls durch Fluor, Chlor, Brom, Cyano, Nitro, C₁-C₂-Alkyl (welches gegebenenfalls durch Fluor und/oder Chlor substituiert ist), C₁-C₂-Alkoxy (welches gegebenenfalls durch Fluor und/oder Chlor substituiert ist), C₁-C₂-Alkylthio (welches gegebenenfalls durch Fluor und/oder Chlor substituiert ist) oder C₁-C₂-Alkylsulfonyl (welches gegebenenfalls durch Fluor und/oder Chlor substituiert ist) substituiert sind,
n für 2, 3 oder 4 steht,
Y für N-CN oder N-NO₂ steht,
Z für S oder NR³ steht und
R³ für Wasserstoff, Methyl, Ethyl, n-Propyl oder i-Propyl steht

3. Verbindungen der Formel (I) gemäß Anspruch 1, worin
A für Thiazolyl oder Pyridyl steht, welche jeweils gegebenenfalls substituiert sind durch Halogen oder C₁-C₃-Alkyl oder
A für einen gegebenenfalls durch Halogen oder C₁-C₃-Alkyl substituierten Tetrahydrofurylrest steht,
R¹ für Wasserstoff oder Methyl steht,
R² für Wasserstoff, Methyl, oder gegebenenfalls durch Halogen, Cyano oder durch jeweils gegebenenfalls durch Fluor oder Chlor substituiertes Methyl, Ethyl, Methoxy oder Ethoxy substituiertes Phenyl oder für Thiazolyl, Pyridyl oder Pyrazinyl steht,
n für 2 oder 3 steht,
Y für NCN oder NNO₂ steht,
Z für S oder NR³ steht und
R³ für Wasserstoff oder Methyl steht.

4. Verbindungen der Formel (I) gemäß Anspruch 1, worin
A für einen der Reste 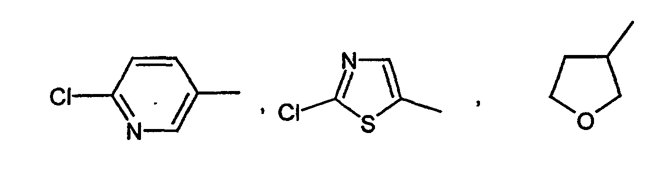 steht,
R¹ für Wasserstoff oder Methyl steht,
R² für Wasserstoff oder Methyl steht,
n für 2 oder 3 steht,
Y für NCN oder
Y für NNO₂ steht,
Z für S oder NR³ steht und
R³ für Wasserstoff oder Methyl steht.

5. Verfahren zur Herstellung von Verbindungen der Formel (I), **dadurch gekennzeichnet, dass** man Verbindungen der Formel (II) 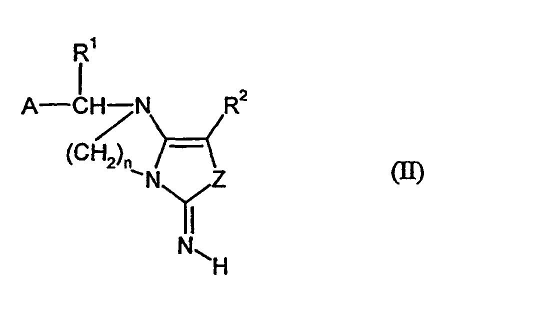 mit geeigneten Cyanierungsreagenzien oder mit geeigneten Nitrierungsreagenzien umsetzt.

6. Mittel zur Bekämpfung tierischer Schädlinge, **dadurch gekennzeichnet, dass** sie mindestens eine Verbindung der Formel (I) gemäß Anspruch 1 enthalten.

7. Verwendung von Verbindungen der Formel (I) gemäß Anspruch 1 zur Bekämpfung tierischer Schädlinge.

8. Herstellung von Mitteln zur Bekämpfung tierischer Schädlinge, **dadurch gekennzeichnet, dass** man Verbindungen der Formel (I) gemäß Anspruch 1 mit Verdünnungsmitteln und/oder oberflächenaktiven Stoffen vermischt.

## Claims

1. Compounds of the formula (I) 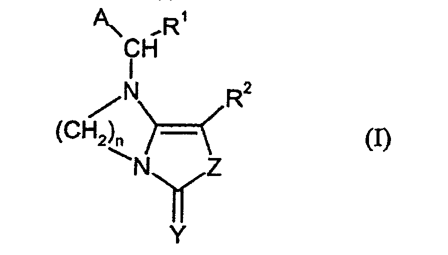 in which
A represents in each case optionally substituted aryl or hetaryl or heterocyclyl,
R¹ represents hydrogen or C₁-C₃-alkyl,
R² represents hydrogen, C₁-C₃-alkyl or in each case optionally substituted aryl or hetaryl,
n represents 2, 3 or 4,
Y represents N-CN or N-NO₂,
Z represents S, SO, SO₂ or NR³ and
R³ represents hydrogen or C₁-C₃-alkyl.

2. Compounds of the formula (I) according to Claim 1, in which
A represents optionally halogen-, cyano-, nitro-, C₁-C₄-alkyl-, C₁-C₄-haloalkyl-, C₁-C₄-alkoxy- or C₁-C₄-haloalkoxy-substituted phenyl or
A represents pyrazolyl, 1,2,4-triazolyl, oxazolyl, isoxazolyl, thiazolyl, isothiazolyl, 1,2,5-thiadiazolyl, pyridyl, pyrazinyl or pyrimidinyl, which are optionally substituted by fluorine, chlorine, bromine, cyano, nitro, C₁-C₂-alkyl (which is optionally substituted by fluorine and/or chlorine), C₁-C₂-alkoxy (which is optionally substituted by fluorine and/or chlorine), C₁-C₂-alkylthio (which is optionally substituted by fluorine and/or chlorine) or C₁-C₂-alkylsulphonyl (which is optionally substituted by fluorine and/or chlorine), or
A represents an optionally halogen- or C₁-C₃-alkyl-substituted saturated C₅-C₆-cycloalkyl radical in which one methylene group is replaced by O or S,
R¹ represents hydrogen, methyl, ethyl, n-propyl or i-propyl,
R² represents hydrogen, methyl, ethyl, n-propyl, i-propyl, represents optionally halogen-, cyano-, nitro-, C₁-C₄-alkyl-, C₁-C₄-haloalkyl-, C₁-C₄-alkoxy- or C₁-C₄-haloalkoxy-substituted phenyl or represents pyrazolyl, 1,2,4-triazolyl, oxazolyl, isoxazolyl, thiazolyl, isothiazolyl, 1,2,5-thiadiazolyl, pyridyl, pyrazinyl or pyrimidinyl which are optionally substituted by fluorine, chlorine, bromine, cyano, nitro, C₁-C₂-alkyl (which is optionally substituted by fluorine and/or chlorine), C₁-C₂-alkoxy (which is optionally substituted by fluorine and/or chlorine), C₁-C₂-alkylthio (which is optionally substituted by fluorine and/or chlorine) or C₁-C₂-alkylsulphonyl (which is optionally substituted by fluorine and/or chlorine),
n represents 2, 3 or 4,
Y represents N-CN or N-NO₂,
Z represents S or NR³ and
R³ represents hydrogen, methyl, ethyl, n-propyl or i-propyl.

3. Compounds of the formula (I) according to Claim 1, in which
A represents thiazolyl or pyridyl, which are each optionally substituted by halogen or C₁-C₃-alkyl or
A represents an optionally halogen- or C₁-C₃-alkyl-substituted tetrahydrofuryl radical,
R¹ represents hydrogen or methyl,
R² represents hydrogen, methyl, or represents phenyl which is optionally substituted by halogen, cyano or by in each case optionally fluorine- or chlorine-substituted methyl, ethyl, methoxy or ethoxy, or represents thiazolyl, pyridyl or pyrazinyl,
n represents 2 or 3,
Y represents NCN or NNO₂,
Z represents S or NR³, and
R³ represents hydrogen or methyl.

4. Compounds of the formula (I) according to Claim 1, in which
A represents one of the radicals 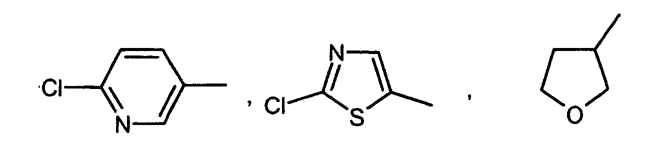
R¹ represents hydrogen or methyl,
R² represents hydrogen or methyl,
n represents 2 or 3,
Y represents NCN,
Y represents NNO₂.
Z represents S or NR³ and
R³ represents hydrogen or methyl.

5. Process for preparing compounds of the formula (I), **characterized in that** compounds of the formula (II) 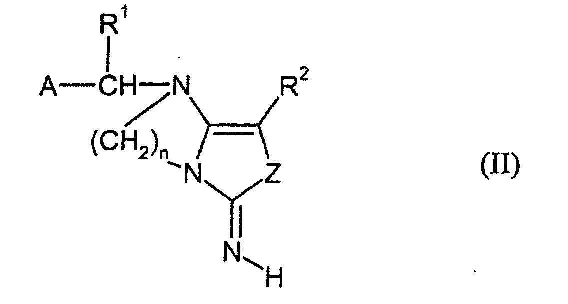 are reacted with suitable cyanating agents or with suitable nitrating agents.

6. Compositions for controlling animal pests, **characterized in that** they comprise at least one compound of the formula (I) according to Claim 1.

7. Use of compounds of the formula (I) according to Claim 1 for controlling animal pests.

8. Preparation of compositions for controlling animal pests, **characterized in that** compounds of the formula (1) according to Claim 1 are mixed with diluents and/or surfactants.

## Revendications

1. Composés de la formule (I) : 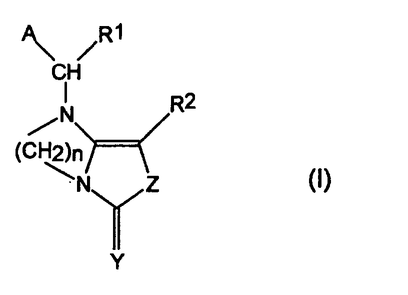 dans laquelle
A représente un radical aryle ou hétéroaryle ou hétérocyclyle, chaque fois le cas échéant substitué ;
R¹ représente l'atome d'hydrogène ou un radical alkyle en C₁-C₃;
R² représente l'atome d'hydrogène, un radical alkyle en C₁-C₃ ou représente un radical aryle ou hétéroaryle, chaque fois le cas échéant substitué;
n représente 2, 3 ou 4 ;
Y représente N-CN ou N-NO₂ ;
Z représente S, SO, SO₂ ou NR³, et
R³ représente l'atome d'hydrogène ou un radical alkyle en C₁-C₃.

2. Composés de la formule (I) suivant la revendication 1, où
A représente le radical phényle le cas échéant substitué par un atome d'halogène, le radical cyano, nitro, un radical alkyle en C₁-C₄, halogénoalkyle en C₁-C₄, alcoxy en C₁-C₄ ou halogénoalcoxy en C₁-C₄, ou
A représente le radical pyrazolyle, 1,2,4-triazolyle, oxazolyle, isoxazolyle, thiazolyle, isothiazolyle, 1,2,5-thiadiazolyle, pyridyle, pyrazinyle ou pyrimidinyle, qui sont le cas échéant, substitués par l'atome de fluor, de chlore, de brome, le radical cyano, nitro, un radical alkyle en C₁-C₂ (qui peut être le cas échéant, substitué par l'atome de fluor et/ou de chlore), alcoxy en C₁-C₂ (qui peut être le cas échéant, substitué par l'atome de fluor et/ou de chlore), alkylthio en C₁-C₂ (qui peut être le cas échéant, substitué par l'atome de fluor et/ou de chlore) ou alkylsulfonyle en C₁-C₂ (qui peut être le cas échéant, substitué par l'atome de fluor et/ou de chlore), ou
A représente un radical cycloalkyle en C₅-C₆ saturé, le cas échéant substitué par un atome d'halogène ou un radical alkyle en C₁-C₃, dans lequel un radical méthylène est remplacé par O ou S ;
R¹ représente l'atome d'hydrogène, le radical méthyle, éthyle, n-propyle ou i-propyle ;
R² représente l'atome d'hydrogène, le radical méthyle, éthyle, n-propyle ou i-propyle, le radical phényle le cas échéant substitué par un atome d'halogène, le radical cyano, nitro, un radical alkyle en C₁-C₄, halogénoalkyle en C₁-C₄, alcoxy en C₁-C₄ ou halogénoalcoxy en C₁-C₄, ou représente le radical pyrazolyle, 1,2,4-triazolyle, oxazolyle, isoxazolyle, thiazolyle, isothiazolyle, 1,2,5-thiadiazolyle, pyridyle, pyrazinyle ou pyrimidinyle, qui sont le cas échéant, substitués par l'atome de fluor, de chlore, de brome, le radical cyano, nitro, un radical alkyle en C₁-C₂ (qui peut être le cas échéant, substitué par l'atome de fluor et/ou de chlore), alcoxy en C₁-C₂ (qui peut être le cas échéant, substitué par l'atome de fluor et/ou de chlore), alkylthio en C₁-C₂ (qui peut être le cas échéant, substitué par l'atome de fluor et/ou de chlore) ou alkylsulfonyle en C₁-C₂ (qui peut être le cas échéant, substitué par l'atome de fluor et/ou de chlore) ;
n représente 2, 3 ou 4 ;
Y représente N-CN ou N-NO₂ ;
Z représente S ou NR³, et
R³ représente l'atome d'hydrogène, le radical méthyle, éthyle, n-propyle ou i-propyle.

3. Composés de la formule (I) suivant la revendication 1, où
A représente le radical thiazolyle ou pyridyle, qui sont le cas échéant, substitués par un atome d'halogène ou un radical alkyle en C₁-C₃, ou
A représente le radical tétrahydrofuryle le cas échéant substitué par un atome d'halogène ou un radical alkyle en C₁-C₃ ;
R¹ représente l'atome d'hydrogène ou le radical méthyle ;
R² représente l'atome d'hydrogène, le radical méthyle, ou le radical phényle le cas échéant substitué par un atome d'halogène, le radical cyano, ou le radical méthyle, éthyle, méthoxy ou éthoxy, chaque fois le cas échéant substitué par l'atome de fluor ou de chlore, ou représente le radical thiazolyle, pyridyle ou pyrazinyle ;
n représente 2 ou 3 ;
Y représente N-CN ou N-NO₂ ;
Z représente S ou NR³, et
R³ représente l'atome d'hydrogène ou le radical méthyle.

4. Composés de la formule (I) suivant la revendication 1, où
A représente un des restes . : 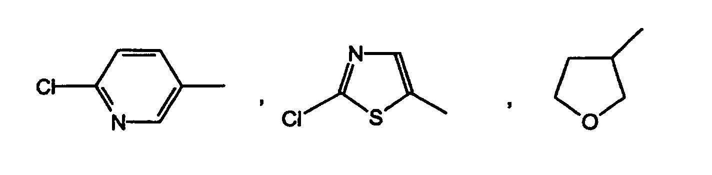 R¹ représente l'atome d'hydrogène ou le radical méthyle ;
R² représente l'atome d'hydrogène ou le radical méthyle ;
n représente 2 ou 3 ;
Y représente N-CN, ou
Y représente N-NO₂ ;
Z représente S ou NR³, et
R³ représente l'atome d'hydrogène ou le radical méthyle.

5. Procédé de préparation des composés de la formule (I), **caractérisé en ce que** l'on fait réagir des composés de la formule (II) : 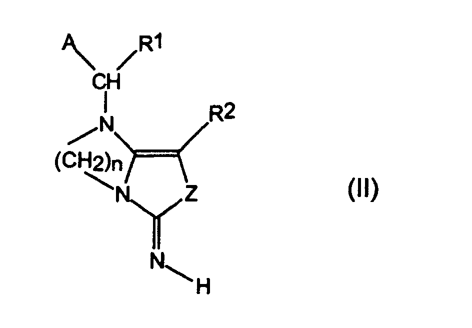 avec des réactifs appropriés de cyanuration ou avec des réactifs appropriés de nitration.

6. Agents pour lutter contre les parasites animaux, **caractérisé en ce qu'**ils contiennent au moins un composé de la formule (I) suivant la revendication 1.

7. Utilisation des composés de la formule (I) suivant la revendication 1, pour lutter contre les parasites animaux.

8. Préparation d'agents pour lutter contre les parasites animaux, **caractérisée en ce que** l'on mélange les composés de la formule (I) suivant la revendication 1 avec des agents de dilution et/ou des substances tensioactives.
